# EUROPEAN PATENT APPLICATION

(11) **EP 4 282 980 A1**
(43) Date of publication of application: **29.11.2023**
(21) Application number: 22174952.6
(22) Date of filing: 23.05.2022
(51) Int. Cl.: C12Q 1/6848

(54) **METHODS FOR AMPLIFYING A NUCLEIC ACID**

(71) Applicant: Mobidiag Oy, 02150 Espoo (FI)
(72) Inventor: Niinivaara, Anne, 02150 Espoo (FI); Saharinen, Juha, 02150 Espoo (FI); Kirveskari, Juha, 02150 Espoo (FI)
(74) Representative: Maiwald GmbH

(57) **Abstract**

Provided is a method for amplifying a nucleic acid which is suspected to be present in a sample in a low copy number comprises the following steps:
i. providing a first reaction mixture in a reaction chamber wherein the first reaction mixture comprises a first portion of the sample;
ii. subjecting the first reaction mixture in the reaction chamber to conditions allowing a pre-amplification of the nucleic acid in order to obtain a first pre-amplification mixture;
iii. providing a second reaction mixture in the reaction chamber wherein the second reaction mixture comprises a subset of the first pre-amplification mixture and a second portion of the sample;
iv. subjecting the second reaction mixture to conditions allowing a pre-amplification of the nucleic acid in order to obtain a second pre-amplification mixture;
v. optionally, repeating steps iii) and iv) a number N additional times in order to obtain an (N+2) pre-amplification mixture;
vi. subjecting the second pre-amplification mixture obtained in step (iv) or the (N+2) pre-amplification mixture obtained in step (v) to conditions allowing an amplification of the nucleic acid in order to obtain an amplification mixture,
wherein the sample has a total sample volume which exceeds a volume capacity of the reaction chamber.

## Description

### FIELD

The present disclosure provides methods for amplifying a nucleic acid suspected to be present in a low copy number in a sample.

### BACKGROUND

Conventional nucleic acid amplification technologies, such as the polymerase chain reaction (PCR), allow the amplification of a target nucleic acid present in a sample. However, in case of sample types with typically high sample volume and/or a suspected low concentration of the target nucleic acid, introduction of a single sample portion or aliquot into a conventional nucleic acid amplification reaction chamber may lead to the absence of any amplification by conventional approaches.

Examples for sample types for which the introduction of a single sample portion or aliquot may be insufficient for amplification by conventional nucleic acid amplification technologies, are sample types that are dilute by nature, such as human or animal tissues or excretions, like urine, stool, cerebrospinal fluid, blood, sputum and bronchoalveolar lavage, and water system samples. To overcome the problem of dilute samples, further processing steps, such as sample concentration or nucleic acid precipitation, are necessary, extending the hands-on processing time and/or requiring additional equipment and reagents. Moreover, it is possible that additional processing still results in a low target analyte concentration and a sample volume exceeding the volume capacity of the reaction chamber.

In addition to naturally dilute samples, sample types, such as stool, sputum, nasopharyngeal aspirates and blood or blood containing tissue samples, require dilution, e.g. to overcome inhibition by amplification reaction inhibitory substances. As a result, a single sample portion or aliquot introduced into the nucleic acid amplification reaction chamber might not contain the target nucleic acid to be amplified if present in a low copy number.

Examples for applications aiming for detection of a target nucleic acid are the detection of microbial DNA, e.g. in blood samples from sepsis patients or the analysis of nasopharyngeal swabs or salvia from suspected SARS-CoV-2 patients containing a low copy number of SARS-CoV-2 RNA. Failed amplification by conventional nucleic acid amplification technologies may result in a false negative detection of the target nucleic acid.

Thus, there is a need for methods that allow the amplification of target nucleic acids suspected to be present in a low copy number.

### BRIEF SUMMARY

Methods for amplifying a nucleic acid suspected to be present in low copy number in a sample with a total sample volume exceeding a volume capacity of a regular nucleic acid amplification reaction chamber are provided.

In a first aspect, a method for amplifying a nucleic acid which is suspected to be present in a sample in a low copy number comprises the following steps:
i. providing a first reaction mixture in a reaction chamber wherein the first reaction mixture comprises a first portion of the sample;
ii. subjecting the first reaction mixture in the reaction chamber to conditions allowing a pre-amplification of the nucleic acid in order to obtain a first pre-amplification mixture;
iii. providing a second reaction mixture in the reaction chamber wherein the second reaction mixture comprises a subset of the first pre-amplification mixture and a second portion of the sample;
iv. subjecting the second reaction mixture to conditions allowing a pre-amplification of the nucleic acid in order to obtain a second pre-amplification mixture;
v. optionally, repeating steps iii) and iv) a number N additional times in order to obtain an (N+2) pre-amplification mixture;
vi. subjecting the second pre-amplification mixture obtained in step (iv) or the (N+2) pre-amplification mixture obtained in step (v) to conditions allowing an amplification of the nucleic acid in order to obtain an amplification mixture,
wherein the sample has a total sample volume which exceeds a volume capacity of the reaction chamber.

According to one embodiment, providing a second reaction mixture in the reaction chamber in step (iii) comprises displacing a subset of the first pre-amplification mixture in the reaction chamber by introducing the second portion of the sample into the reaction chamber.

A volume of the second portion of the sample introduced into the reaction chamber can be 1-99% of the volume of the first pre-amplification mixture, optionally 10-95%, 20-90%, 30-80% or 40 to 70% or 50-60% of the volume of the first pre-amplification mixture.

According to another embodiment, providing a second reaction mixture in the reaction chamber in step (iii) comprises removing a subset of the first pre-amplification mixture from the reaction chamber in order to provide a remaining subset of the first pre-amplification mixture in the reaction chamber and combining the remaining subset of the first pre-amplification mixture with a second portion of the sample in order to provide the second reaction mixture.

According to another embodiment, providing a second reaction mixture in the reaction chamber in step (iii) comprises removing a subset of the first pre-amplification mixture from the reaction chamber, combining the removed subset of the first pre-amplification mixture with a second portion of the sample in order to provide a combined pre-amplification sample mixture and introducing a portion of the combined pre-amplification sample mixture into the reaction chamber in order to provide the second reaction mixture.

In another aspect, a method for amplifying a nucleic acid which is suspected to be present in a sample in a low copy number comprises the following steps:
i. providing a first reaction mixture in a reaction chamber wherein the first reaction mixture comprises a first portion of the sample;
ii. subjecting the first reaction mixture in the reaction chamber to conditions allowing a pre-amplification of the nucleic acid in order to obtain a first pre-amplification mixture;
iii. removing a subset of the first pre-amplification mixture from the reaction chamber in order to provide a remaining subset of the first pre-amplification mixture in the reaction chamber;
iv. providing a second reaction mixture in the reaction chamber wherein the second reaction mixture comprises the remaining subset of the first pre-amplification mixture and a second portion of the sample;
v. subjecting the second reaction mixture to conditions allowing a pre-amplification of the nucleic acid in order to obtain a second pre-amplification mixture;
vi. optionally, repeating steps iii) to v) a number N additional times in order to obtain an (N+2) pre-amplification mixture;
vii. subjecting the second pre-amplification mixture obtained in step (v) or the (N+2) pre-amplification mixture obtained in step (vi) to conditions allowing an amplification of the nucleic acid in order to obtain an amplification mixture,
wherein the sample has a total sample volume which exceeds a volume capacity of the reaction chamber.

In another aspect, a method for amplifying a nucleic acid which is suspected to be present in a sample in a low copy number comprises the following steps:
i. providing the sample in a sample chamber;
ii. transferring a first portion of the sample to a reaction chamber in order to obtain a first reaction mixture;
iii. subjecting the first reaction mixture in the reaction chamber to conditions allowing a pre-amplification of the nucleic acid in order to obtain a first pre-amplification mixture;
iv. transferring at least a subset of the first pre-amplification mixture from the reaction chamber to the sample chamber comprising a second portion of the sample in order to provide a combined pre-amplification sample mixture;
v. transferring a portion of the combined pre-amplification sample mixture to the reaction chamber in order to provide the second reaction mixture;
vi. subjecting the second reaction mixture to conditions allowing a pre-amplification of the nucleic acid in order to obtain a second pre-amplification mixture;
vii. optionally, repeating steps iv) to vi) a number N additional times in order to obtain an (N+2) pre-amplification mixture;
viii. subjecting the second pre-amplification mixture obtained in step (v) or the (N+2) pre-amplification mixture obtained in step (vi) to conditions allowing an amplification of the nucleic acid in order to obtain an amplification mixture,
wherein the sample has a total sample volume which exceeds a volume capacity of the reaction chamber.

In some embodiments of the above methods, the volume of the subset of the first pre-amplification mixture removed from the reaction chamber or transferred from the reaction chamber to the sample chamber is 1-99% of the volume of the pre-amplification mixture, optionally 25-95%, 30-90%, 40-80%, 50-70% or 55-65% or about 60% of the volume of the pre-amplification mixture.

In some embodiments of the above methods, the total sample volume exceeds the volume capacity of the reaction chamber by at least 110%, optionally at least 150%, at least 200% or at least 1000%.

In some embodiments of the above methods, the volume capacity of the reaction chamber is 10-100 µl, optionally 20-90 µl, 30-80 µl, 40-70 µl or 50-60 µl.

In some embodiments of the above methods, the total sample volume is 100-500 µl, optionally 150-450 µl or 200-400 µl or 250-350 µl or about 300 µl.

In some embodiments of the above methods, the average amount of nucleic acid in the sample is less than 25 copies per 50 µl sample volume, optionally less than 15 copies or less than 5 copies per 50 µl sample volume or 1 or 2 copies per 50 µl sample volume or even less, e.g. 1 or 2 copies per 100 µl sample volume or 1 or 2 copies per 1 ml sample volume.

In some embodiments of the above methods, the volume of the first reaction mixture is substantially identical to a volume of the second reaction mixture

In some embodiments of the above methods, both the pre-amplification and the amplification are a cyclic amplification. Alternatively, both the pre-amplification and the amplification are an isothermal amplification. In other embodiments of the above methods the pre-amplification is a cyclic amplification and the amplification is an isothermal amplification, or the pre-amplification is an isothermal amplification and the amplification is a cyclic amplification.

In some embodiments of the above methods, the pre-amplification is a cyclic amplification with 3-15 cycles, optionally 3-12 cycles or 4-11 cycles or 4-10 cycles or 4-8 cycles or 4-6 cycles.

In some embodiments of the above methods, the pre-amplification is an isothermal amplification of 5 to 30 min, optionally 5 to less than 30 min, optionally 10 to 25 min or 15 to 20 min.

In some embodiments of the above methods, the amplification is a cyclic amplification having 50 cycles or less, optionally 25-45 cycles or 30 to 40 cycles or about 35 cycles.

In some embodiments of the above methods, the amplification is an isothermal amplification of 20 to 60 min, optionally 25 to 60 min, optionally 30 to 60 min or 30 to 55 min or 35 to 50 min or 40 to 45 min.

In some embodiments of the above methods, N is sufficient to subject substantially the total sample volume to conditions allowing a pre-amplification of the nucleic acid.

In some embodiments of the above methods, the first and second reaction mixture comprise one or more of a DNA-dependent DNA polymerase, a DNA-dependent RNA polymerase, and an RNA-dependent DNA polymerase.

For example, the DNA-dependent DNA polymerase is selected from a *Taq* polymerase, SuperFi DNA Polymerase, AccuPrime *Pfx* DNA Polymerase, *Pfu* polymerase, deep vent polymerase, *Pwo* polymerase, *Tli* polymerase, *Tfu* polymerase, T4 DNA polymerase, T7 DNA polymerase, and Q5 DNA polymerase. An exemplary DNA-dependent RNA polymerase can be selected from a T7 RNA Polymerase; T3 RNA Polymerase, SP6 RNA Polymerase, Poly (A)- or Poly (U)-polymerase, and Hi-T7 RNA Polymerase An exemplary RNA-dependent DNA polymerase can be elected from a Moloney Murine Leukemia Virus Reverse Transcriptase (M-MLV RT), Avian Myeloblastosis Virus Reverse Transcriptase (AMV RT), ProScript Reverse Transcript, and SuperScript reverse transcriptase.

In some embodiments of the above methods, subjecting to conditions allowing an amplification comprises adding further amplification reagents wherein said further amplification reagents comprise one or more of a DNA-dependent DNA polymerase, a DNA-dependent RNA polymerase, and an RNA-dependent DNA polymerase. In some embodiments, the amplification reagents added comprise a DNA-dependent DNA polymerase selected form a *Taq* polymerase, SuperFi DNA Polymerase, AccuPrime *Pfx* DNA Polymerase, *Pfu* polymerase, deep vent polymerase, *Pwo* polymerase, *Tli* polymerase, *Tfu* polymerase, T4 DNA polymerase, T7 DNA polymerase, and Q5 DNA polymerase. In other embodiments, the amplification reagents added comprise a DNA-dependent RNA polymerase selected from a T7 RNA Polymerase; T3 RNA Polymerase, SP6 RNA Polymerase, Poly (A)- or Poly (U)-polymerase, and Hi-T7 RNA Polymerase. In other embodiments, the amplification reagents added comprise RNA-dependent DNA polymerase selected from a Moloney Murine Leukemia Virus Reverse Transcriptase (M-MLV RT), Avian Myeloblastosis Virus Reverse Transcriptase (AMV RT), ProScript Reverse Transcript, and SuperScript reverse transcriptase.

In another embodiment of the above methods, the method comprises a further step of determining in the amplification reaction mixture a value indicative for the presence and/or amount of the nucleic acid present in the sample.

In some embodiments of the above methods, the nucleic acid is indicative for a disease selected from the group consisting of a respiratory disease, COVID-19, a gastrointestinal disease and sepsis.

In another aspect, a method for amplifying a nucleic acid which is suspected to be present in a sample in a low copy number comprises the following steps:
i. providing a first reaction mixture in a reaction chamber wherein the first reaction mixture comprises a first portion of the sample;
ii. subjecting the first reaction mixture in the reaction chamber to conditions allowing a pre-amplification of the nucleic acid in order to obtain a first pre-amplification mixture;
iii. providing a second reaction mixture in the reaction chamber wherein the second reaction mixture comprises a subset of the first pre-amplification mixture and a second portion of the sample;
iv. subjecting the second reaction mixture to conditions allowing an amplification of the nucleic acid in order to obtain an amplification mixture,
wherein the sample has a total sample volume which exceeds a volume capacity of the reaction chamber

In some embodiments of the above methods, one or more of the steps are performed in a microfluidic cartridge.

### BRIEF DESCRIPTION OF THE SEVERAL VIEWS OF THE DRAWINGS

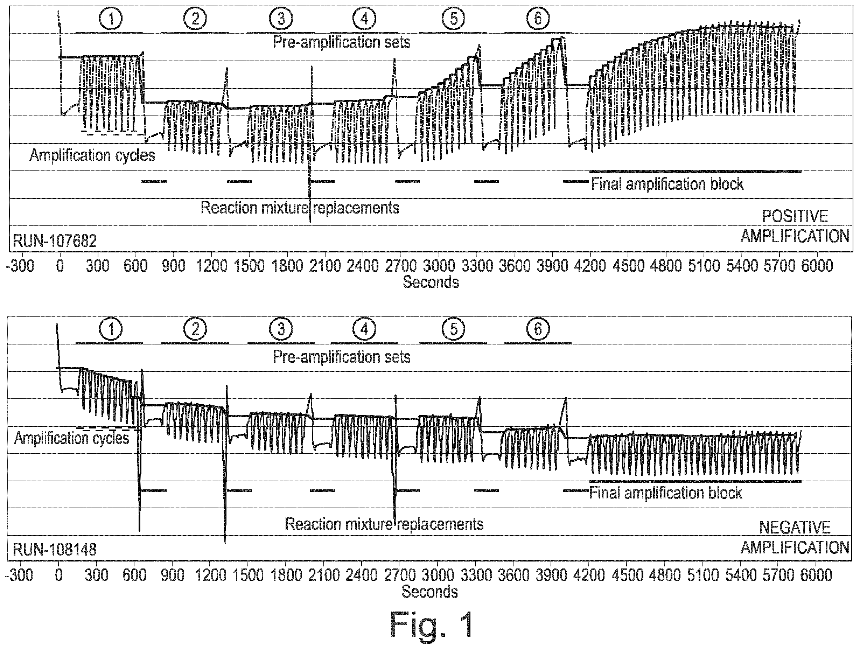

Figure 1 shows results of an exemplary method of the present disclosure in which a target nucleic acid is detected using qPCR. This exemplary method is performed using 6 pre-amplifications (N = 4), wherein each pre-amplification is a cyclic pre-amplification with 10 cycles, which are followed by an amplification of 35 cycles. The volume capacity of the reaction chamber is 20 µl. After each pre-amplification, 10 µl of the amplification mixture is removed and another sample portion of 10 µl is inserted. Thus, a total sample volume of 70 µl is subjected to conditions allowing pre-amplification of the target nucleic acid. The presence of the target nucleic acid is assessed in the amplification step using qPCR. In the upper panel, the sample is a dilute 10 cfu/ml bacterial sample solution. A clear detection with apparent quantification cycle (Cq ∼ cycle) 15 is observed in the amplification curves shown, illustrating the increased sensitivity achieved with the method of the present disclosure. In the lower panel, no target nucleic acid is present in the sample resulting in a negative amplification.

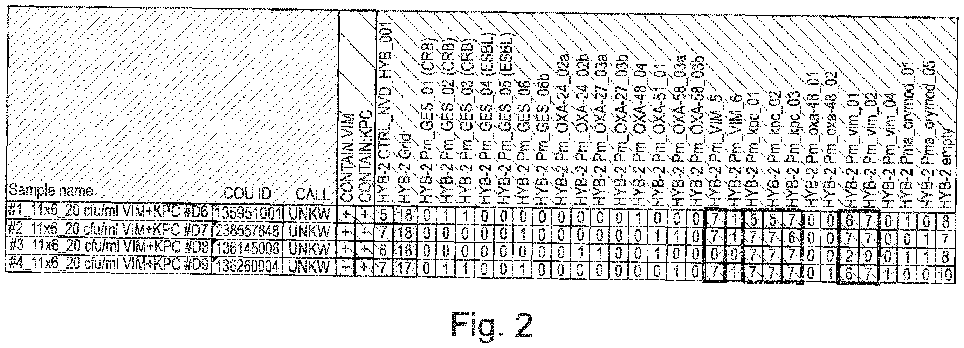

Figure 2 shows results of another exemplary method of the present disclosure including a hybridization detection. In this example, a sample concentration of 20 cfu/ml of carbapenemase gene containing *P. aeruginosa* and *E. cloacae* bacteria as targets (VIM and KPC, respectively) was used. As can be seen, 4 out of 4 replicates for KPC targets and 3 out of 4 of the replicates for VIM targets indicated a positive detection. The exemplary method was performed with 6 (N = 4) pre-amplification steps each having 11 cycles. Pm_VIM_01/02/5 designates probes immobilized to a biochip which are complementary to the (amplified) VIM target. Pm_kpc_01/02/03 designates probes immobilized to a biochip which are complementary to the (amplified) KPC target.

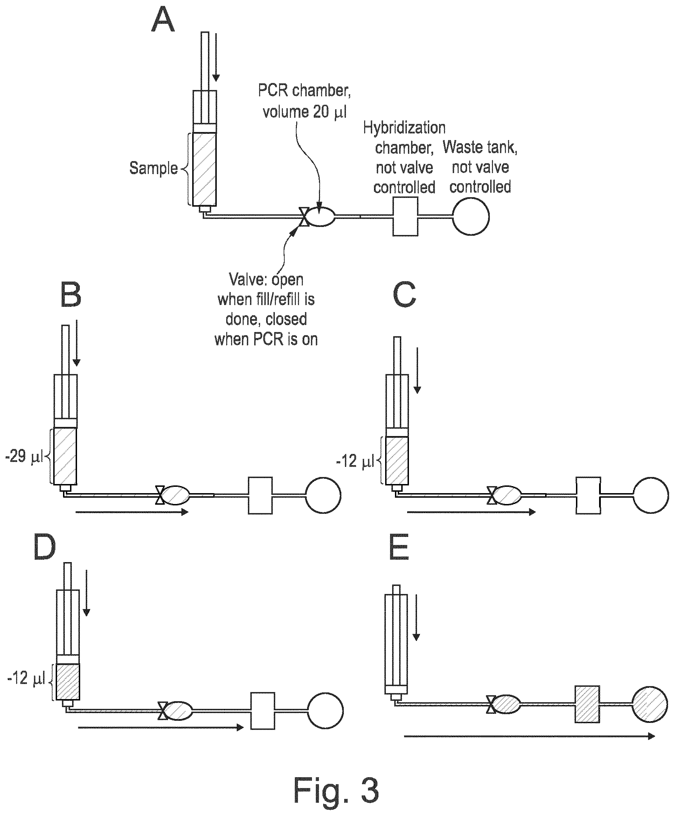

Figure 3 shows a schematic overview of an exemplary experimental setup of the method of the present disclosure. A) Shows the starting position prior to subjecting the sample to an exemplary method of the present disclosure. The sample, present in a syringe, contains all reagents necessary for amplification and is ready to be infused portion by portion. B) In a first step, the syringe infuses a first sample portion, which is sufficient to fill the 20 µl reaction chamber. The first pre-amplification is performed, resulting in a first pre-amplification mixture. C) In a second step, the syringe infuses 12 µl of a second portion of the sample, pushing or displacing a subset or part of the first pre-amplification mixture towards a waste chamber. A second pre-amplification is performed, resulting in a second pre-amplification mixture. D) In a third step, the syringe infuses 12 µl of a third portion of the sample, pushing or displacing a subset or part of the second pre-amplification mixture towards the waste chamber. E) In a next step or series of steps, the total sample volume is processed portion by portion through the reaction chamber. The amplicons that accumulated in the reaction chamber are exponentially amplified in a final amplification of 25 cycles producing an amplification mixture which is then transferred to a hybridization chamber.

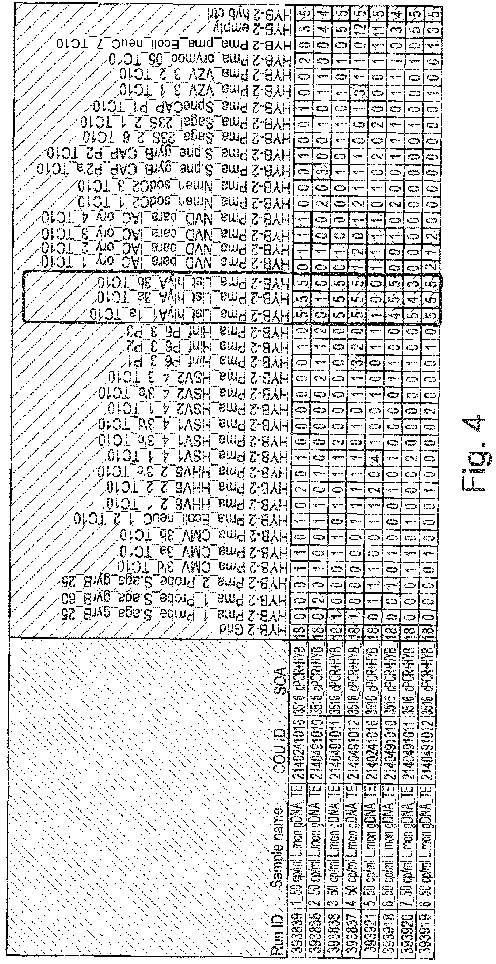

Figure 4 shows results of the exemplary method of the present disclosure as described in Example 3. In brief, a sample of 150 µl with a concentration of 50 cp/ml *of L. monocytogenes* gDNA was mixed with 150 µl of amplification reagents. 221 µl of the resulting sample volume (one reaction chamber filling of 29 µl, followed by 16 reaction chamber fillings of 12 µl) were subjected to a total of 16 pre-amplifications (N =14) of 4 cycles each and a final amplification of 25 cycles. The presence of the target nucleic acid was determined using hybridization end-point detection. The figure shows that out of 8 cartridges in which the method of the present disclosure was performed (shown as rows), 6 resulted in the positive detection of the target of *L. monocytogenes* nucleic acid (fields highlighted in green within the area surrounded by a black circle). Thus, the probability of detection was 75%.

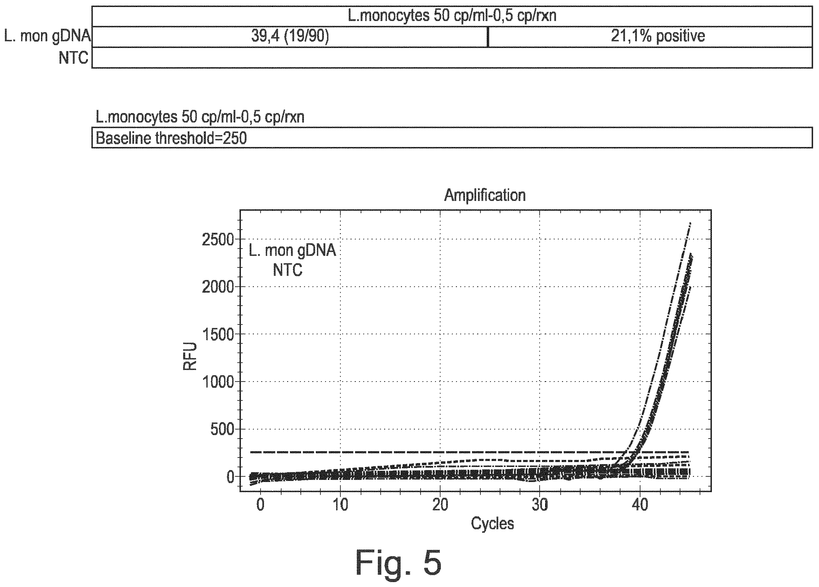

Figure 5 shows results of a Biorad CFX qPCR run with 90 replicates of the same sample as a comparison with the method of the present disclosure as described in Example 3. The reaction volume of each replicate was 20 µl including 10 µl of a sample with a concentration of 50 cp/ml of *L. monocytogenes* gDNA. The figure shows that only in 19 out of 90 reaction chambers, a positive amplification signal was detected (21.1% probability of detection).

### DETAILED DESCRIPTION

Terms used in the claims and specification are defined as set forth below unless otherwise specified.

The term "comprising" is used herein to mean "including, but not necessarily limited to".

It is noted that as used herein, the singular forms "a," "an," and "the" include plural referents unless the context clearly dictates otherwise. Thus, the articles "a", "an" and "the" are used herein to refer to one or to more than one (e.g. to "one or more" or "at least one") of the grammatical object of the article. Thus, for example, reference to "a nucleic acid" includes one nucleic acid or a plurality of such nucleic acids and reference to "the nucleic acid" includes reference to one or more nucleic acids and equivalents thereof known to those skilled in the art, and so forth.

Where a range of values is provided, it is understood that each intervening value, to the tenth of the unit of the lower limit unless the context clearly dictates otherwise, between the upper and lower limit of that range and any other stated or intervening value in that stated range, is encompassed within this disclosure. The upper and lower limits of these smaller ranges may independently be included in the smaller ranges, and are also encompassed within this disclosure, subject to any specifically excluded limit in the stated range. Where the stated range includes one or both of the limits, ranges excluding either or both of those included limits are also included in the disclosure.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are described. All publications mentioned herein are incorporated herein by reference to disclose and describe the methods and/or materials in connection with which the publications are cited.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable sub-combination. All combinations of the embodiments pertaining to the disclosure are specifically embraced by the present disclosure and are disclosed herein just as if each and every combination was individually and explicitly disclosed. In addition, all sub-combinations of the various embodiments and elements thereof are also specifically embraced by the present disclosure and are disclosed herein just as if each and every such sub-combination was individually and explicitly disclosed herein.

As used herein, the term "sample" is used in its broadest sense. Typically, a sample within the context of the present disclosure is suspected of comprising a target sequence, gene or template, e.g., the presence or absence of which can be determined using the methods of the present disclosure. For example, a sample may be meant to include a specimen or culture (e.g., microbiological culture), or it may be meant to include both biological and environmental samples (e.g., suspected of comprising a target sequence, gene or template nucleic acid). A sample may include a specimen of synthetic origin. Samples may be unpurified or may be partially or completely purified or otherwise processed, e.g. by extracting, precipitating or diluting, and may be further enriched or concentrated.

The present disclosure is not limited by the type of biological sample used or analyzed. The present disclosure is useful with a variety of biological samples including, but are not limited to, tissue (e.g., organ (e.g., heart, liver, brain, lung, stomach, intestine, spleen, kidney, pancreas, and reproductive (e.g., ovaries) organs), glandular, skin, and muscle tissue), cell (e.g., blood cell (e.g., lymphocyte or erythrocyte), muscle cell, tumor cell, and skin cell), gas, bodily fluid (e.g., blood or portion thereof, serum, plasma, urine, semen, saliva, cerebrospinal fluid, pleural fluid, milk, lymph, sputum, needle aspirates, bronchoalveolar lavage, etc.), or solid (e.g., stool) samples obtained from a human (e.g., adult, infant, or embryo) or animal (e.g., cattle, poultry, mouse, rat, dog, pig, cat, horse, and the like). Biological samples may be solid food and/or feed products and/or ingredients such as dairy items, vegetables, meat and meat by-products, and waste. Biological samples may be obtained from all of the various families of domestic animals, as well as feral or wild animals, including, but not limited to, such animals as ungulates, bear, fish, lagomorphs, rodents, etc.

Biological samples also include biopsies and tissue sections (e.g., biopsy or section of tumor, growth, rash, infection, or paraffin-embedded sections), medical or hospital samples (e.g., including, but not limited to, blood samples, saliva, buccal swab, cerebrospinal fluid, bronchoalveolar lavage, needle aspirates, pleural fluid, milk, colostrum, lymph, sputum, vomitus, bile, semen, oocytes, cervical cells, amniotic fluid, urine, stool, hair and sweat), laboratory samples (e.g., subcellular fractions), and forensic samples (e.g., blood or tissue (e.g., spatter or residue), hair and skin cells containing nucleic acids), and archeological samples (e.g., fossilized organisms, tissue, or cells).

Environmental samples include, but are not limited to, environmental material such as surface matter, soil, water (e.g., freshwater or seawater), algae, lichens, geological samples, air containing materials containing nucleic acids, crystals, and industrial samples, as well as samples obtained from food and dairy processing instruments, apparatus, equipment, utensils, disposable and non-disposable items.

Other types of biological samples may contain bacteria (e.g., Actinobacteria (e.g., Actinomyces, Arthrobacter, Corynebacterium (e.g., *C. diphtheriae)),* Mycobacterium (e.g., *M. tuberculosis* and *M. leprae),* Propionibacterium (e.g., *P. acnes),* Streptomyces, Chlamydiae (e.g., C. *trachomatis* and C. *pneumoniae),* Cyanobacteria, Deinococcus (e.g., Thermus (e.g., *T. aquaticus)),* Firmicutes (e.g., Bacilli (e.g., *B. anthracis, B. cereus, B. thuringiensis,* and *B. subtilis)),* Listeria (e.g., *L. monocytogenes),* Staphylococcus (e.g., S. *aureus, S. epidermidis,* and S. *haemolyticus),* Streptococcus, Enterococcus, Fusobacteria, Proteobacteria (e.g., Rickettsiales, Sphingomonadales, Bordtella (e.g., *B. pertussis),* Neisserisales (e.g., *N. gonorrhoeae* and *N. meningitidis),* Enterobacteriales (e.g., Escherichia (e.g., *E. coli),* Klebsiella, Plesiomonas, Proteus, Salmonella, Shigella, and Yersinia and Enterobacter (e.g. *E. cloacae)),* Legionellales, Pasteurellales (e.g., *Haemophilus influenzae),* Pseudomonas, Vibrio (e.g., *V. cholerae* and V. *vulnificus), Pseudomonas aeruginosa,* Campylobacterales (e.g., Campylobacteria (e.g., *C. jejuni),* and Helicobacter (e.g., *H. pylori)),* and Spirochaetes (e.g., Leptospira, *B. bergdorferi*, and *T. pallidum));* Archaea (e.g., Halobacteria and Methanobacteria); Eucarya (e.g., Animalia (e.g., Annelidia, Arthropoda (e.g., Chelicerata, Myriapoda, Insecta, and Crustacea), Mollusca, Nematoda, (e.g., *C. elegans,* and *T. spiralis)* and Chordata (e.g., Actinopterygii, Amphibia, Aves, Chondrichthyes, Reptilia, and Mammalia (e.g., Primates, Rodentia, Lagomorpha, and Carnivora)))); Fungi (e.g., Dermatophytes, Fusarium, Penicillum, Candidas, Cryptococcus, Aspergillus, and Saccharomyces); Plantae (e.g., Magnoliophyta (e.g., Magnoliopsida and Liliopsida)), and Protista (e.g., Apicomplexa (e.g., Cryptosporidium, Plasmodium (e.g., P. falciparum, and Toxoplasma), and Metamonada (e.g., *G. lambia*))).

Other types of biological samples may contain viruses (e.g., dsDNA viruses (e.g., Bacteriophage, Adenoviridae, Herpesviridiae, Papillomaviridae, Polyomaviridae, and Poxyiridae), ssDNA viruses (e.g., Parvoviridae), dsRNA viruses (including Reoviridae), (+)ssRNA viruses (e.g., Coronaviridae, Astroviridae, Bromoviridae, Comoviridae, Flaviviridae, Picornaviridae, and Togaviridae), (-) ssRNA viruses (e.g., Bornaviridae, Filoviridae, Paramyxoviridae, Rhabdoviridae, Bunyaviridae, and Orthomyxovirdiae), ssRNA-reverse transcribing viruses (e.g., Retroviridae), and dsDNA-reverse transcribing viruses (e.g., Hepadnaviridae and Caulomoviridae)).

The sample may be prepared by any desired or suitable method. Nucleic acids may be analyzed directly from bodily fluids or other samples using the methods described in U.S. Pat. Pub. Serial No. 20050186588, herein incorporated by reference in its entirety.

The above described examples are not, however, to be construed as limiting the sample types applicable to the present disclosure.

A preferred sample is a DNA or RNA sample isolated or purified from a biological sample. Another preferred sample is a sample containing microbial cells such as bacterial cells isolated from a complex biological sample.

In this description, the term "sample" can also refer to a mixture of any of the above-mentioned samples with reagents suitable for nucleic acid amplification reaction, preferably "amplification reagents" as defined below.

The term "bacteria" refers to any bacterial species.

The terms "archaea," "archaeal species," "archaean" and "archaebacteria" are used interchangeably refer to any organisms classified as a member of the Archaea domain or kingdom of life.

The term "virus" refers to obligate, ultramicroscopic, intracellular parasites incapable of autonomous replication (e.g, replication requires the use of the host cell's machinery)

The term "nucleic acid" or "nucleic acid molecule" as used herein refer to an oligonucleotide, nucleotide or polynucleotide, and fragments or portions thereof. A nucleic acid may be a DNA or RNA, of either genomic or synthetic origin, that may be single or double stranded, and represent the sense or antisense strand. Thus, nucleic acids may be dsDNA, ssDNA, mixed ssDNA, mixed dsDNA, dsDNA made into ssDNA (e.g., through melting, denaturing, helicases, etc.), A-, B-, or Z- DNA, triple-stranded DNA, R A, ssRNA, dsRNA, mixed ss and dsRNA, dsRNA made into ssRNA (e.g. , via melting, denaturing, helicases, etc.), messenger RNA (mRNA), ribosomal RNA (rRNA), transfer RNA (tRNA), catalytic RNA, snRNA, microRNA, or protein nucleic acid (PNA).

The terms "nucleotide" and "base" are used interchangeably when used in reference to a nucleic acid sequence, unless indicated otherwise herein.

As used herein, "target nucleic acid" refers to a region of a nucleic acid that is to be amplified, and/or detected. The "target nucleic acid" can reside between two primer sequences used for amplification.

As used herein, the term "complementary" refers to the capacity for precise pairing between two nucleotides; e.g, if a nucleotide at a given position of a nucleic acid is capable of hydrogen bonding with a nucleotide of another nucleic acid to form a canonical base pair, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. The degree of complementarity between nucleic acid strands can have significant effects on the efficiency and strength of hybridization between nucleic acid strands.

Nucleic acids of the present disclosure may encompass sequences that include analogs of DNA and RNA nucleotides, including, but not limited to, 4-acetylcytosine, 8-hydroxy-N6-methyladenosine, aziridinylcytosine, pseudoisocytosine, 5-(carboxyhydroxyl-methyl) uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudo-uracil, 1-methylguanine, 1-methylinosine, 2,2-dimethyl-guanine, 2-methyladenine, 2-methylguanine, 3-methyl-cytosine, 5-methylcytosine, N6-methyladenine, 7-methylguanine, 5-methylaminomethyluracil, 5-methoxy-aminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxy acetic acid, oxybutoxosine, pseudouracil, queosine, 2-thiocytosine, 5-methyl-2-thiouracil, 2-thiouracil, 4-thiouracil, 5-methyluracil, N-uracil-5-oxy acetic acid methylester, uracil-5-oxyacetic acid, pseudouracil, queosine, 2-thiocytosine, 2,6-diaminopurine, and pyrazolo[3,4-d]pyrimidines such as guanine analogue 6 amino 1H-pyrazolo[3,4d]pyrimidin 4(5H) one (ppG or PPG, also Super G) and the adenine analogue 4 amino 1H-pyrazolo[3,4d]pyrimidine (ppA or PPA).

In addition to the modified bases noted above, nucleic acids of the disclosure can have a backbone of sugar or glycosidic moieties, preferably 2-deoxyribofuranosides wherein all internucleotide linkages are the naturally occurring phosphodiester linkages. The 2-deoxy-P-D-ribofuranose groups may be replaced with other sugars, for example, β-D-ribofuranose. In addition, β-D-ribofuranose may be present wherein the 2-OH of the ribose moiety is alkylated with a Ci_6 alkyl group (2-(0-Ci_6 alkyl) ribose) or with a C_{2_}6 alkenyl group (2-(0- C₂₋₆ alkenyl) ribose), or is replaced by a fluoro group (2-fluororibose). Related oligomer-forming sugars useful in the present disclosure are those that are "locked", e.g , contain a methylene bridge between C-4' and an oxygen atom at C-2'. Other sugar moieties compatible with hybridization of the oligonucleotide can also be used, and are known to those of skill in the art, including, but not limited to, α-D-arabinofuranosides, a-2'-deoxyribofuranosides or 2',3'-dideoxy-3'-aminoribofuranosides. Oligonucleotides containing a-D-arabinofuranosides can be prepared as described in U.S. Pat. No. 5, 177, 196. Oligonucleotides containing 2', 3'-dideoxy-3'-aminoribofuranosides are described in Chen et al. Nucleic Acids Res. 23 :2661-2668 (1995). Synthetic procedures for locked nucleic acids (Singh et al, Chem. Comm., 455-456 (1998); Wengel J., Acc. Chem. Res., 32:301-310 (1998)) and oligonucleotides containing 2'-halogen-2'-deoxyribofuranosides (Palissa et al, Z. Chem., 27:216 (1987)) have also been described. The phosphate backbone of the modified oligonucleotides described herein can also be modified so that the oligonucleotides contain phosphorothioate linkages and/or methylphosphonates and/or phosphoroamidates (Chen et al, Nucl. Acids Res., 23 :2662-2668 (1995)). Combinations of oligonucleotide linkages are also within the scope of the present disclosure. Still other backbone modifications are known to those of skill in the art.

In the context of the present description, the term "amplicon" means the product of a nucleic acid amplification reaction. Amplicons may be produced by a variety of amplification reactions whose products are multiple replicates of the sequence of one or more target nucleic acids.

The term "amplified" as used herein refers to an increase in the abundance of nucleic acid. A target nucleic acid may be amplified, e.g., by *in vitro* replication such as by PCR or by TMA.

"Nucleic acid amplification" refers to any *in vitro* procedure that produces multiple copies of a target nucleic acid sequence, or its complementary sequence, or fragments thereof (e.g, an amplified sequence containing less than the complete target nucleic acid). Examples of nucleic acid amplification procedures include transcription associated methods, such as transcription-mediated amplification (TMA), nucleic acid sequence-based amplification (NASBA) and others (e.g., US Pat. Nos. 5,399,491, 5,554,516, 5,437,990, 5,130,238, 4,868,105, and 5,124,246), replicase-mediated amplification (e.g., US Pat. No. 4,786,600), the polymerase chain reaction (PCR), including real-time PCR and reverse transcription PCR (e.g., US Pat. Nos. 4,683,195, 4,683,202, and 4,800,159), ligase chain reaction (LCR) (e.g., EP Pat. App. 0320308), helicase-dependent amplification (e.g., US Pat. No. 7,282,328), and strand-displacement amplification (SDA) (e.g., US Pat. No. 5,422,252).

Amplification may be linear or exponential.

Replicase-mediated amplification uses self-replicating RNA molecules, and a replicase such as QB-replicase. PCR amplification uses DNA polymerase, primers, and thermocycling steps to synthesize multiple copies of the two complementary strands of DNA or cDNA. LCR amplification uses at least four separate oligonucleotides to amplify a target and its complementary strand by using multiple cycles of hybridization, ligation, and denaturation. Helicase-dependent amplification uses a helicase to separate the two strands of a DNA duplex generating single-stranded templates, followed by hybridization of sequence-specific primers hybridize to the templates and extension by DNA polymerase to amplify the target sequence. SDA uses a primer that contains a recognition site for a restriction endonuclease that will nick one strand of a hemi-modified DNA duplex that includes the target sequence, followed by amplification in a series of primer extension and strand displacement steps.

Amplification can be performed by cycling through two or three temperature points, referred to as thermocycling and is then also referred to as a cyclic amplification. Amplification can also be performed by holding at a single temperature point, referred to as isothermal amplification.

One example for a cyclic amplification method is PCR. Traditional PCR methods include the following steps: denaturation or melting of double-stranded nucleic acids; annealing of primers to the denatured single-stranded nucleic acids; and extension of the primers using a polymerase to form double-stranded nucleic acids. These steps are each performed at a different temperature point. This cycle is repeated by denaturing at the denaturation step. The number of copies of the target sequence in principle grows exponentially. In practice, it typically doubles with each cycle until reaching a plateau at which more primer-template accumulates than the enzyme can extend during the cycle; then the increase in target nucleic acid becomes linear.

A reverse transcription PCR method includes an initial reverse transcription step to produce cDNA from RNA, and then otherwise follows the general steps listed above for PCR.

Transcription associated amplification uses a reverse transcriptase, a DNA polymerase, an RNA polymerase, deoxyribonucleoside triphosphates, ribonucleoside triphosphates, a promoter-containing oligonucleotide, and optionally may include other oligonucleotides, to ultimately produce multiple RNA transcripts from a nucleic acid template (described in detail in US Pat. Nos. 5,399,491 and 5,554,516, Kacian et al., US Pat. No. 5,437,990, Burg et al., PCT Nos. WO 88/01302 and WO 88/10315, Gingeras et al., US Pat. No. 5,130,238, Malek et al., US Pat. Nos. 4,868,105 and 5,124,246, Urdea et al., PCT No. WO 94/03472, McDonough et al., PCT No. WO 95/03430, and Ryder et al.). Methods that use TMA are described in detail previously (US Pat. Nos. 5,399,491 and 5,554,516).

One example of an isothermal amplification technology is TMA. Amplification methods that use TMA amplification include the following steps. Briefly, a single stranded target nucleic acid containing the target sequence to be amplified is provided. Those skilled in the art will appreciate that conventional melting of double stranded nucleic acid may be used to provide single-stranded target nucleic acids. A first amplification oligomer is brought in contact with that target nucleic acid by hybridizing to the target sequence. The first amplification oligomer may be a primer or a promoter primer. A suitable nucleic acid polymerase then generates a nucleic acid strand amplification product that is complementary to the target nucleic acid target sequence. In the instances where the target nucleic acid is an RNA, the RNA is typically degraded leaving just the newly generated amplification product, which is available for hybridization by a second amplification oligomer. Using a primer as the first amplification oligomer, then the second amplification oligomer is a promoter primer or promoter provider. A suitable nucleic acid polymerase uses the newly generated amplification product to which the promoter-based oligomer is hybridized as a primer to make a complementary strand of the unhybridized promoter sequence. If the second amplification oligomer is a promoter primer, then a complementary copy of the amplification product hybridized by the second amplification oligomer is also generated. The now double stranded promoter sequence of the promoter-based amplification is used by a suitable RNA polymerase to initiate transcription and make RNA transcript amplification products. The first amplification oligomer primer can then hybridize the transcribed amplification products and the steps can repeat. Or, the target nucleic acid is RNA and the first amplification oligomer is a promoter-based amplification oligomer. Here, the promoter based amplification oligomer is a promoter primer. A suitable polymerase makes a first amplification product that is complementary to the RNA target sequence. The RNA target nucleic acid is degraded and a second amplification oligomer is hybridized to the amplification product. A suitable polymerase makes a complement strand, thereby generating a double stranded promoter sequence. Transcription is initiated and RNA is transcribed. The transcribed RNA is complementary to the original target nucleic acid, thus the second amplification oligomer hybridizes again and makes the transcribed RNA double stranded. The RNA is degraded and the remaining DNA strand is hybridized by the first amplification oligomer. The amplification steps can repeat. When the target nucleic acid is DNA the first amplification oligomer is a promoter primer and the second amplification is a primer. Amplification generally proceeds as described above, and as is described in the art. See e.g., US Pat. Nos. 4,868,105; 5,124,246; 5,130,238; 5,399,491; 5,437,990; 5,554,516; and 7,374,885; and PCT Pub. Nos. WO 88/01302; WO 88/10315 and WO 95/03430 describing TMA and other variations of transcription-associated amplification. The amplified products may be detected in real-time during amplification, or at the end of the amplification reaction. Detection may be performed by a number of methods. Probe-based detection methods use an oligonucleotide probe comprising a target hybridizing sequence that binds specifically to a target sequence contained in the amplification products. Detection of a signal resulting from the bound probes indicates the presence of the target nucleic acid in the sample.

A "reagent" refers broadly to any agent used in a reaction, other than the analyte (e.g., target nucleic acid being analyzed).

The term "amplification reagents" as used herein, refers to all the necessary reagents for performing an amplification reaction, which may include, but not be limited to, a polymerase (e.g. DNA-dependent DNA polymerase, DNA-dependent RNA polymerase , RNA-dependent DNA polymerase), a helicase, a nuclease (e.g. endonuclease), a replicase such as QB-replicase, target-specific oligonucleotides (e.g. primers, probes), dNTPs, NTPs, buffering agents to maintain pH at a selected level during a reaction, salts, co-factors, scavengers, labels, dyes, and the like.

A "reaction mixture" as used herein, including a first reaction mixture, a second reaction mixture or an (N+2) reaction mixture, comprises all amplification reagents necessary to allow for the amplification of the nucleic acid for which amplification is sought, e.g the target nucleic acid. A reaction mixture may include, but not be limited to, one or more of a target nucleic acid, a polymerase (e.g. DNA-dependent DNA polymerase, DNA-dependent RNA polymerase , RNA-dependent DNA polymerase), a helicase, a nuclease (e.g. endonuclease), a replicase such as QB-replicase, target-specific oligonucleotides (e.g. primers, probes), dNTPs, NTPs, buffering agents to maintain pH at a selected level during a reaction, salts, co-factors, scavengers, labels, dyes, and the like.

As used herein, a "DNA-dependent DNA polymerase" is an enzyme that synthesizes a complementary DNA copy from a DNA template. DNA-dependent DNA polymerases may be the naturally occurring enzymes isolated from bacteria or bacteriophages or expressed recombinantly. Also, DNA-dependent DNA polymerases may be modified forms which have been engineered to possess certain desirable characteristics, e.g., thermostability, or the ability to recognize or synthesize a DNA strand from various modified templates. All known DNA-dependent DNA polymerases require a complementary primer to initiate synthesis. It is known that under suitable conditions a DNA-dependent DNA polymerase may synthesize a complementary DNA copy from an RNA template. Exemplary DNA-dependent DNA polymerases include: *Taq* polymerase, SuperFi DNA Polymerase *(Taq* polymerase engineered to have proofreading characteristics), AccuPrime *Pfx* DNA Polymerase *(Pfx* polymerase with proofreading and hot-start capabilities), *Pfu* polymerase, deep vent polymerase, *Pwo* polymerase, *Tli* polymerase, *Tfu* polymerase, T4 DNA polymerase, T7 DNA polymerase, and Q5 DNA polymerase. DNA-dependent DNA polymerases are commercially available (see, e.g., New England BioLabs, Ipswich, MA; Thermo Fisher Scientific, Waltham, MA; and Promega Corporation, Madison, WI).

As used herein, a "DNA-dependent RNA polymerase" or "transcriptase" is an enzyme that synthesizes multiple RNA copies from a double-stranded or partially double-stranded DNA molecule having a promoter sequence that is usually double-stranded. The RNA molecules ("transcripts") are synthesized in the 5'-to-3' direction beginning at a specific position just downstream of the promoter. Transcriptases can be naturally occurring, recombinantly expressed, wild-type, or engineered. Exemplary transcriptases include, T7 RNA Polymerase; T3 RNA Polymerase, SP6 RNA Polymerase, Poly (A)- or Poly (U)-polymerase (engineered to provide a Poly(A) or Poly(U) tale on RNA product), and Hi-T7 RNA Polymerase (engineered to operate at higher temperatures than wild-type T7 RNA polymerase). DNA-dependent RNA polymerases are commercially available (see, e.g., New England BioLabs, Ipswich, MA; Thermo Fisher Scientific, Waltham, MA; and Promega Corporation, Madison, WI).

As used herein, an "RNA-dependent DNA polymerase" or "reverse transcriptase" ("RT") is an enzyme that synthesizes a complementary DNA copy from an RNA template. Reverse transcriptases can be naturally occurring, recombinantly expressed, wild-type, or engineered. Many reverse transcriptases also can make a complementary DNA copy from a DNA template; thus, they are both RNA- and DNA-dependent DNA polymerases. RTs may also have an RNAse H activity. A primer is required to initiate synthesis with both RNA and DNA templates. Exemplary reverse transcriptases include: Moloney Murine Leukemia Virus Reverse Transcriptase (M-MLV RT), Avian Myeloblastosis Virus Reverse Transcriptase (AMV RT), ProScript Reverse Transcript (engineered to have reduced RNAse activity), and SuperScript reverse transcriptase (engineered to operate at higher temperatures and to possess faster reaction speed). RNA-dependent RNA polymerases are commercially available (see, e.g., New England BioLabs, Ipswich, MA; Thermo Fisher Scientific, Waltham, MA; and Promega Corporation, Madison, WI).

The term "target-specific oligonucleotide" or "primer" or "probe", as used herein is defined as a molecule comprising two or more nucleotides (e.g., deoxyribonucleotides or ribonucleotides), preferably at least 5 nucleotides, more preferably at least about 10-15 nucleotides and more preferably at least about 15 to 30 nucleotides, or longer (e.g. , oligonucleotides are typically less than 200 residues long (e.g., between 15 and 100 nucleotides), however, as used herein, the term is also intended to encompass longer polynucleotide chains). The exact size will depend on many factors, which in turn depend on the ultimate function or use of the oligonucleotide. Target specific oligonucleotides are often referred to by their length. For example a 24 residue oligonucleotide is referred to as a "24-mer". Target specific oligonucleotides can form secondary and tertiary structures by self-hybridizing or by hybridizing to other polynucleotides. Such structures can include, but are not limited to, duplexes, hairpins, cruciforms, bends, and triplexes.

Target-specific oligonucleotides may be generated in any manner, including chemical synthesis, DNA replication, reverse transcription, PCR, or a combination thereof. Preferably, target-specific oligonucleotides of the present disclosure are synthesized using solid phase methods. Standard phosphoramidite solid-phase chemistry for joining nucleotides by phosphodiester linkages is disclosed by Caruthers et al., in "Chemical Synthesis of Deoxynucleotides by the Phosphoramidite Method," Methods Enzymol., 154:287 (1987). Automated solid-phase chemical synthesis using cyanoethyl phosphoramidite precursors has been described by Barone. See Barone et al., "In Situ Activation of bis-dialkylaminephosphines--a New Method for Synthesizing Deoxyoligonucleotides on Polymer Supports," Nucleic Acids Res., 12(10):4051(1984). Moreover, methods for the organic synthesis of oligonucleotides are known to those of skill in the art and are described in, for example, Sambrook et al., supra, ch. 10.

Target-specific oligonucleotides may be modified in any way, as long as a given modification is compatible with the desired function of a given oligonucleotide. One of ordinary skill in the art can easily determine whether a given modification is suitable or desired for any given oligonucleotide of the present disclosure. Modifications include, but are not limited to base modifications, sugar modifications and backbone modifications.

A target-specific oligonucleotide can comprise a label, preferably a detectable label. A target-specific oligonucleotide comprising a detectable label can be used as a probe or as a primer, for example, as described herein. Various labels can be introduced into a target-specific oligonucleotide. As one of ordinary skill in the art will appreciate, the location of a label within a target-specific oligonucleotide of the present disclosure can vary and is not limited to the disclosure herein.

The term "label," as used herein, refers to any atom or molecule that can be used to provide a detectable and/or quantifiable signal. In particular, the label can be attached, directly or indirectly, to a nucleic acid or protein. Suitable labels that can be attached to probes include, but are not limited to, radioisotopes, fluorophores, chromophores, mass labels, electron dense particles, magnetic particles, spin labels, molecules that emit chemiluminescence, electrochemically active molecules, enzymes, cofactors, and enzyme substrates.

A label may be joined, directly or indirectly, to an oligonucleotide or other biological molecule. Direct labeling can occur through bonds or interactions that link the label to the oligonucleotide, including covalent bonds or non-covalent interactions such as hydrogen bonding, hydrophobic and ionic interactions, or through formation of chelates or coordination complexes. Indirect labeling can occur through use of a bridging moiety or "linker", such as an antibody or additional oligonucleotide(s), which is/are either directly or indirectly labeled.

The term "dye," as used herein, generally refers to any organic or inorganic molecule that absorbs electromagnetic radiation at a wavelength greater than or equal 300 nm. The term "fluorescent dye," as used herein, generally refers to any dye that emits electromagnetic radiation of longer wavelength by a fluorescent mechanism upon irradiation by a source of electromagnetic radiation, such as a lamp, a photodiode, or a laser or another fluorescent dye.

The term "real time" as used herein in reference to detection of nucleic acid amplification or signal amplification refers to the detection or measurement of the accumulation of products or signal in the reaction while the reaction is in progress, e.g., during incubation or thermocycling. Such detection or measurement may occur continuously, or it may occur at a plurality of discrete points during the progress of the amplification reaction, or it may be a combination. For example, in a polymerase chain reaction, detection (e.g., of fluorescence) may occur continuously during all or part of thermocycling, or it may occur transiently, at one or more points during one or more cycles. Real time detection of PCR can accomplished by determining a level of fluorescence at the same point (e.g., a time point in the cycle, or temperature step in the cycle) in each of a plurality of cycles, or in every cycle. Real time detection of amplification may also be referred to as detection "during" the amplification reaction. Similarly, an isothermal amplification reaction may be a real-time reaction with continuous or discrete point detection of amplification product (see e.g., WO 2014/018885).

The term "qPCR" generally refers to the PCR technique known as real-time quantitative polymerase chain reaction, quantitative polymerase chain reaction or kinetic polymerase chain reaction. This technique simultaneously amplifies and quantifies target nucleic acids using PCR wherein the quantification is by virtue of an intercalating fluorescent dye or sequence-specific probes which contain fluorescent reporter molecules that are only detectable once hybridized to a target nucleic acid.

The data from a qPCR experiment can be depicted as growth curves in which the number of cycles required for a detectable signal (such as fluorescence intensity from fluorescent dyes) to cross background (threshold) level, which is commonly referred as the Cycle threshold or "Ct value". Similarly, an isothermal amplification reaction may be a quantitative reaction (see e.g., WO 2018/075633).

The term "reaction chamber" or "nucleic acid amplification reaction chamber" as used herein refers to any chamber or vessel or container or well which is suitable for accommodating a sample or portion or aliquot of the sample for the purpose of a nucleic acid amplification. A reaction chamber as used in the methods of the present disclosure has a conventional volume capacity, e.g. in the range of from 10 to 200 µl, or 10 to 150 µl or 10 to 100 µl or 10 to 50 µl or 15 to 40 µl, or 20 to 30 µl. A volume capacity of the reaction chamber means a volume of the reaction chamber configured to accommodate a liquid for subjecting it to a nucleic acid amplification reaction.

A "microfluidic cartridge" or "microfluidic device" means an integrated system of one or more chambers, ports, and channels that are interconnected and in fluid communication and designed for carrying out an analytical reaction or process, either alone or in cooperation with an appliance or instrument that provides support functions, such as sample introduction, fluid and/or reagent driving means, temperature control, and a detection system. Microfluidics may further include valves, pumps, and specialized functional coatings on their interior walls, e.g. to prevent adsorption of sample components or reactants, facilitate reagent movement by electroosmosis, or the like. Such devices are usually fabricated in or as a solid substrate, which may be glass, plastic, or other solid polymeric materials, and typically have a planar format for ease of detecting and monitoring sample and reagent movement, especially via optical or electrochemical methods. Features of a microfluidic device usually have cross-sectional dimensions of less than a few hundred square micrometers and passages typically have capillary dimensions, e.g. having maximal cross-sectional dimensions of from about 500 mm to about 0.1 mm. Microfluidics devices typically have volume capacities in the range of from 20 ml to a 1 µl or lower, e.g. 10 µl-15 ml or 100 µl to 1 ml. The fabrication and operation of microfluidics devices are well-known in the art.

In a first aspect, this disclosure provides a method for amplifying a nucleic acid which is suspected to be present in a sample in a low copy number, wherein the method comprises the following steps:
i. providing a first reaction mixture in a reaction chamber wherein the first reaction mixture comprises a first portion of the sample;
ii. subjecting the first reaction mixture in the reaction chamber to conditions allowing a pre-amplification of the nucleic acid in order to obtain a first pre-amplification mixture;
iii. providing a second reaction mixture in the reaction chamber wherein the second reaction mixture comprises a subset of the first pre-amplification mixture and a second portion of the sample;
iv. optionally, subjecting the second reaction mixture to conditions allowing a pre-amplification of the nucleic acid in order to obtain a second pre-amplification mixture;
v. optionally, repeating steps iii) and iv) a number N additional times in order to obtain an (N+2) pre-amplification mixture;
vi. subjecting the second reaction mixture of step iii), the second pre-amplification mixture obtained in step (iv) or the (N+2) pre-amplification mixture obtained in step (v) to conditions allowing an amplification of the nucleic acid in order to obtain an amplification mixture,
wherein the sample has a total sample volume which exceeds a volume capacity of the reaction chamber.

Providing a first reaction mixture in a reaction chamber may comprise transferring a first portion of the sample from a sample chamber which accommodates the sample to the reaction chamber.

To facilitate the processing of a large sample volume, a subset of the pre-amplification mixture may be removed prior to the insertion of another sample portion. Thus, in some embodiments, providing a second reaction mixture in the reaction chamber comprises removing a subset of the first pre-amplification mixture from the reaction chamber in order to provide a remaining subset of the first pre-amplification mixture in the reaction chamber and combining the remaining subset of the first pre-amplification mixture with a second portion of the sample in order to provide the second reaction mixture. In this embodiment, the removed subset of the first pre-amplification mixture can e.g. be transferred to a waste chamber or be transferred to a store chamber for later processing.

Providing a second reaction mixture in the reaction chamber wherein the second reaction mixture comprises a subset of the first pre-amplification mixture and a second portion of the sample may also be performed in a flow-through mode.

In an exemplary embodiment, the reaction chamber is filled with the second portion of the sample thereby displacing a subset of the first pre-amplification mixture from the reaction chamber. In other words, a second portion of the sample is introduced into the reaction chamber or transferred from the sample chamber to the reaction chamber thereby displacing a subset of the pre-amplification mixture, e.g. into a waste chamber or store chamber. A remaining subset of the pre-amplification mixture with possible pre-amplified amplicons together with the second portion of the sample can then be subjected to the next pre-amplification step in the reaction chamber.

Providing a second reaction mixture in the reaction chamber in step (iii) may thus comprise displacing a subset of the first pre-amplification mixture in the reaction chamber by the second portion of the sample. The volume of the subset of the first pre-amplification mixture displaced in step (iii) by introducing a second portion of the sample can be 1-99% of the volume of the pre-amplification mixture, optionally 25-90%, 30-80%, 40-70% or 50 to 60% of the volume of the first pre-amplification mixture.

Providing a second reaction mixture in the reaction chamber in step (iii) may also comprise displacing a subset of the first pre-amplification mixture in the reaction chamber by the second portion of the sample, wherein a volume of the second portion of the sample introduced into the reaction chamber is 1-99% of the volume of the first pre-amplification mixture, optionally 10-95%, 20-90%, 30-80% or 40 to 70% or 50-60% of the volume of the first pre-amplification mixture.

In another embodiment, steps iii) and iv) are repeated a number N additional times in order to obtain an (N+2) pre-amplification mixture. The method may then comprise providing a (N+2) reaction mixture in the reaction chamber wherein the (N+2) reaction mixture comprises a subset of the (N+1) pre-amplification mixture and a (N+2) portion of the sample; and subjecting the (N+2) reaction mixture to conditions allowing a pre-amplification of the nucleic acid in order to obtain a (N+2) pre-amplification mixture. For example, if N=1, the method comprises providing a third reaction mixture in the reaction chamber wherein the third reaction mixture comprises a subset of the second pre-amplification mixture and a third portion of the sample; and subjecting the third reaction mixture to conditions allowing a pre-amplification of the nucleic acid in order to obtain a third pre-amplification mixture.

In a specific aspect, this disclosure thus provides a method for amplifying a nucleic acid which is suspected to be present in a sample in a low copy number, wherein the method comprises the following steps:
i. providing a first reaction mixture in a reaction chamber wherein the first reaction mixture comprises a first portion of the sample;
ii. subjecting the first reaction mixture in the reaction chamber to conditions allowing a pre-amplification of the nucleic acid in order to obtain a first pre-amplification mixture;
iii. removing a subset of the first pre-amplification mixture from the reaction chamber in order to provide a remaining subset of the first pre-amplification mixture in the reaction chamber;
iv. providing a second reaction mixture in the reaction chamber wherein the second reaction mixture comprises the remaining subset of the first pre-amplification mixture and a second portion of the sample;
v. subjecting the second reaction mixture to conditions allowing a pre-amplification of the nucleic acid in order to obtain a second pre-amplification mixture;
vi. optionally, repeating steps iii) to v) a number N additional times in order to obtain an (N+2) pre-amplification mixture,
vii. subjecting the second pre-amplification mixture obtained in step (v) or the (N+2) pre-amplification mixture obtained in step (vi) to conditions allowing an amplification of the nucleic acid in order to obtain an amplification mixture,
wherein the sample has a total sample volume which exceeds a volume capacity of the reaction chamber.

In other embodiments, providing a second reaction mixture in the reaction chamber comprises removing a subset of the first pre-amplification mixture from the reaction chamber, combining the removed subset of the first pre-amplification mixture with the second portion of the sample in order to provide a combined pre-amplification sample mixture and introducing a portion of the combined pre-amplification sample mixture into the reaction chamber in order to provide the second reaction mixture.

For example, the removed subset of the first pre-amplification mixture can e.g. be transferred to the sample chamber accommodating a remaining or second portion of the sample, e.g. the portion which was not provided in the reaction chamber in step (i) and has not yet been subjected to conditions allowing a pre-amplification of the nucleic acid. In this exemplary embodiment, after combining the removed subset of the first pre-amplification mixture with the second portion of the sample in the sample chamber, a portion of the combined pre-amplification sample mixture is then transferred from the sample chamber into the reaction chamber in order to provide the second reaction mixture.

In another specific aspect, this disclosure thus provides a method for amplifying a nucleic acid which is suspected to be present in a sample in a low copy number, wherein the method comprises the following steps:
i. providing the sample in a sample chamber;
ii. transferring a first portion of the sample to a reaction chamber in order to obtain a first reaction mixture;
iii. subjecting the first reaction mixture in the reaction chamber to conditions allowing a pre-amplification of the nucleic acid in order to obtain a first pre-amplification mixture;
iv. transferring at least a subset of the first pre-amplification mixture from the reaction chamber to the sample chamber comprising a second portion of the sample in order to provide a combined pre-amplification sample mixture;
v. transferring a portion of the combined pre-amplification sample mixture to the reaction chamber in order to provide the second reaction mixture
vi. subjecting the second reaction mixture to conditions allowing a pre-amplification of the nucleic acid in order to obtain a second pre-amplification mixture;
vii. optionally, repeating steps iv) and vi) a number N additional times in order to obtain an (N+2) pre-amplification mixture,
viii. subjecting the second pre-amplification mixture obtained in step (vi) or the (N+2) pre-amplification mixture obtained in step (vii) to conditions allowing an amplification of the nucleic acid in order to obtain an amplification mixture,
wherein the sample has a total sample volume which exceeds a volume capacity of the reaction chamber.

The above methods generally allow an amplification of target nucleic acids from a total sample volume which exceeds a volume capacity of the reaction chamber.

As used herein, the term "copy number" refers to the amount of a particular target nucleic acid per unit of volume (e.g., 1000 copies per ml).

While the methods of the present disclosure can be applied to sample types containing the target nucleic acid in all ranges of copy numbers, the disclosure is particularly useful for sample types suspected to contain the target nucleic acid in a low copy number. Also, the present disclosure is particularly useful for situations in which the target nucleic acid is suspected to be present in a low copy number in at least a fraction of samples.

For example, nasopharyngeal swabs or salvia from some SARS-CoV-2 patients may contain a high concentration of the target SARS-CoV-2 RNA molecule while it may be present only in low or very low copy number in nasopharyngeal swabs or salvia from other SARS-CoV-2 patients. Conventional nucleic acid amplification methods may result in false negative detection of the target RNA molecule in samples in which it is present in low copy number. Consequently, patients with said low sample concentration of the SARS-CoV-2 RNA molecule may be mistakenly classified as "negative for SARS-CoV-2". The provided methods allow the detection in samples with both high and low concentration, resulting in improved numbers of true positive SARS-CoV-2 RNA detection and correct classification of the patient's SARS-CoV-2 status.

In some embodiments, the amount of nucleic acid in the sample is less than 25 copies per 50 µl. In other embodiments, the amount of nucleic acid in the sample is less than 15 copies. In still other embodiments, the average amount of nucleic acid in the sample is less than 5 copies per 50 µl or 1 or 2 copies per 50 µl sample volume or even less, e.g. 1 or 2 copies per 100 µl sample volume or 1 or 2 copies per 1 ml sample volume.

The present disclosure provides methods in which a large sample volume can be subjected to a nucleic acid amplification reaction in the reaction chamber by subsequently introducing another portion or aliquot of the sample into the reaction chamber.

Thus, the present disclosure is particularly useful for sample types with sample volumes exceeding the volume capacity of a nucleic acid reaction chamber. Examples for sample types for which the present disclosure is particularly useful are samples types that are dilute by nature, such as human/animal tissues/excretions, like urine, cerebrospinal fluid, blood, sputum and bronchoalveolar lavage, and water system samples. Other examples are sample types requiring dilution to overcome inhibition by amplification reaction inhibitory substances, such as stool, sputum, nasopharyngeal aspirates and blood or blood containing tissue samples. Other samples types oftentimes exceeding the volume capacity of a reaction chamber are elution fractions from nucleic acid extraction or purification.

In some embodiments the total sample volume exceeds the volume capacity of the reaction chamber by at least 110%. In some embodiments, the total sample volume exceeds the volume capacity of the reaction chamber by at least 150%. In some embodiments the total sample volume exceeds the volume capacity of the reaction chamber by at least 200%. In some embodiments the total sample volume exceeds the volume capacity of the reaction chamber by at least 1000% or more, e.g. 1500% or 2000%.

The volume capacity of a regular reaction chamber is typically in the low µl range. Thus, in certain embodiments, the volume capacity of the reaction chamber is 10-70 µl. In some embodiments, the volume capacity of the reaction chamber is 20-50 µl. In some embodiments, the volume capacity of the reaction chamber is 30-40 µl.

In some embodiments, the total sample volume is 100-500µl. In some embodiments, the total sample volume is 150-450 µl. In some embodiments, the total sample volume is 200-400 µl. In some embodiments, the total sample volume is 250 to 350 µl. In some embodiments, the total sample volume is about 300 µl.

The amplification reagents may be provided by pre-mixing or contacting the sample with amplification reagents such that each sample portion contains all necessary reagents for the amplification of the target nucleic acid, thereby forming a reaction mixture which is subsequently transferred to the reaction chamber.

The amplification reagents may also be provided by separately transferring a portion of the sample and a portion of the amplification reagents to the reaction chamber, resulting in the formation of a reaction mixture in the reaction chamber.

The reaction mixture may also be provided by contacting the sample portion with the amplification regents upon insertion of the sample portion into the reaction chamber. The first and second reaction mixture typically comprise identical target specific oligonucleotides, e.g. primers. Thus, the nucleic acid to be amplified in all pre-amplification steps, whenever present, is the same.

In some embodiments, the volume of the subset of the first pre-amplification mixture removed from the reaction chamber is 25-95% of the volume of the first pre-amplification mixture. In some embodiments, the volume of the subset of the first pre-amplification mixture removed from the reaction chamber is 30-90% of the volume of the pre-amplification mixture. In some embodiments, the volume of the subset of the first pre-amplification mixture removed from the reaction chamber is 40-80% of the volume of the pre-amplification mixture. In some embodiments, the volume of the subset of the first pre-amplification mixture removed from the reaction chamber is 50-70% of the volume of the pre-amplification mixture. In some embodiments, the volume of the subset of the first pre-amplification mixture removed from the reaction chamber is 55-65% or about 60% of the volume of the pre-amplification mixture.

In some embodiments, if a subset of the first pre-amplification mixture is transferred from the reaction chamber to the sample chamber, the volume of the subset of the first pre-amplification mixture transferred from the reaction chamber to the sample chamber is 1-100%, optionally 50-99% or 60-95% of the volume of the first pre-amplification mixture.

In some embodiments a volume of the first reaction mixture is substantially identical to a volume of the second reaction mixture

The pre-amplification can be a cyclic amplification, such as a PCR. Alternatively, the pre-amplification can be an isothermal amplification.

The purpose of the pre-amplification is to amplify the nucleic acid, whenever present in a reaction mixture, to increase in the abundance of the target nucleic acid molecule. The abundance of a nucleic acid after a pre-amplification step might not be sufficient to allow for direct detection of the nucleic acid without further amplification in step (vi). However, the abundance of a nucleic acid after a pre-amplification step is sufficient to allow for amplification in a further pre-amplification or the amplification in step (vi), even if a substantial portion of the pre-amplification mixture is removed prior to said further amplification or combined with the remaining sample which has not yet been subjected to a pre-amplification.

It will be appreciated by those skilled in the art that the cycle number in cyclic pre-amplifications, and/or the incubation time in isothermal pre-amplifications, respectively, may need to be optimized based on the properties of the individual amplification reaction, such as the length and GC content of the nucleic acid molecule to be amplified.

In a preferred embodiment, the pre-amplification is a cyclic amplification with a cycle number which is lower than the cycle number of a typical cyclic amplification reaction. Exemplary cycle numbers of a cyclic pre-amplification within the context of the present disclosure are 3-15 cycles, optionally 3-12 cycles or 4-11 cycles or 4-10 cycles or 4-8 cycles or 4 to 6 cycles. In another preferred embodiment, the pre-amplification is an isothermal amplification of 5 to 30 min, optionally 5 to less than 30 min, optionally 10 to 25 min or 15 to 20 min.

In some embodiments, the first and second reaction mixture and any optional (N+2) reaction mixture comprise one or more of a DNA-dependent DNA polymerase, a DNA-dependent RNA polymerase, and an RNA-dependent DNA polymerase. In some embodiments, the first and second reaction mixture comprise a DNA-dependent DNA polymerase selected form a *Taq* polymerase, SuperFi DNA Polymerase, AccuPrime *Pfx* DNA Polymerase, *pfu* polymerase, deep vent polymerase, *Pwo* polymerase, *Tli* polymerase, *Tfu* polymerase, T4 DNA polymerase, T7 DNA polymerase, and Q5 DNA polymerase.In some embodiments, the first and second reaction mixture comprise a DNA-dependent RNA polymerase selected from a T7 RNA Polymerase; T3 RNA Polymerase, SP6 RNA Polymerase, Poly (A)- or Poly (U)-polymerase, and Hi-T7 RNA Polymerase. In some embodiments, the first and second reaction mixture comprise a RNA-dependent DNA polymerase selected from a Moloney Murine Leukemia Virus Reverse Transcriptase (M-MLV RT), Avian Myeloblastosis Virus Reverse Transcriptase (AMV RT), ProScript Reverse Transcript, and SuperScript reverse transcriptase.

It will be appreciated by those skilled in the art that the number of pre-amplifications (N +2) may depend on prior or empirical knowledge of the sample and individual preferences. For instance, subjecting the total sample volume or substantially the total sample volume to pre-amplifications may be preferred if the nucleic acid to be amplified is suspected to be present in a very low copy number or little prior knowledge of the sample is available. Thus, in some embodiments, N is sufficient to subject substantially the total sample volume to conditions allowing a pre-amplification of the nucleic acid.

In other situations, the copy number of the target nucleic acid may be suspected to be less than one nucleic acid per volume introduced into the reaction chamber but sufficiently high enough to not require subjecting the entire sample volume to pre-amplification reactions. In such situations, the interest in lowering the total reaction time might outweigh the benefit of subjecting the total sample volume to pre-amplification reactions. Hence, N may be chosen to be sufficient to subject from 20-95% of the sample volume, e.g. 30%, 40%, 50%, 60%, 70%, 80% or 90% of the sample volume, to pre-amplification reactions.

The amplification may be a cyclic amplification, such as a PCR. Alternatively, the amplification may be an isothermal amplification.

The purpose of the amplification is to generate a sufficient number of amplicons to allow for detection of the target nucleic acid in the amplification mixture whenever the amplicon is present in the last pre-amplification mixture obtained prior to the amplification step, e.g. the first or second or (N+2) pre-amplification mixture.

In some embodiments, the amplification is a cyclic amplification having the cycle number of a typical cyclic amplification reaction. Exemplary cycle numbers of a cyclic amplification within the context of the present disclosure are 50 cycles or less, optionally 25-45 cycles or 30 to 40 cycles or about 35 cycles.

In other embodiments, the amplification is an isothermal amplification with a reaction time of a typical isothermal amplification. Exemplary reaction times of a isothermal amplification within the context of the present disclosure are 20 to 60 min, optionally 25 to 60 min, optionally 30 to 60 min or 30 to 55 min or 35 to 50 min or 40 to 45 min.

In some embodiments, both the pre-amplification and the amplification are a cyclic amplification.

Typically, the cycle number of a cyclic pre-amplification is lower than the cycle number of a cyclic amplification.

In other embodiments, both the pre-amplification and the amplification are an isothermal amplification

Typically, the time of an isothermal pre-amplification is shorter than the time of an isothermal amplification.

In still other embodiments, the pre-amplification is a cyclic amplification and the amplification is an isothermal amplification.

In still other embodiments, the pre-amplification is an isothermal amplification and the amplification is a cyclic amplification.

Additional amplification reagents may be added prior to the amplification. These additional amplification reagents may be the same or different amplification reagents as used in the pre-amplification. In some embodiments, subjecting to conditions allowing an amplification comprises adding further amplification reagents wherein said further amplification reagents comprise one or more of a DNA-dependent DNA polymerase, a DNA-dependent RNA polymerase, and an RNA-dependent DNA polymerase. In some embodiments, the added amplification reagents comprise a DNA-dependent DNA polymerase selected form a *Taq* polymerase, SuperFi DNA Polymerase, AccuPrime *Pfx* DNA Polymerase, *pfu* polymerase, deep vent polymerase, *Pwo* polymerase, *Tli* polymerase, *Tfu* polymerase, T4 DNA polymerase, T7 DNA polymerase, and Q5 DNA polymerase. In other embodiments, the added amplification comprise a DNA-dependent RNA polymerase selected from a T7 RNA Polymerase; T3 RNA Polymerase, SP6 RNA Polymerase, Poly (A)- or Poly (U)-polymerase, and Hi-T7 RNA Polymerase. In other embodiments, the added amplification reagents comprise a RNA-dependent DNA polymerase selected from a Moloney Murine Leukemia Virus Reverse Transcriptase (M-MLV RT), Avian Myeloblastosis Virus Reverse Transcriptase (AMV RT), ProScript Reverse Transcript, and SuperScript reverse transcriptase. Also, a further sample portion or aliquot may be introduced into the reaction chamber prior to amplification.

In a preferred embodiment, the method further comprises determining in the amplification reaction mixture a value indicative for the presence and/or amount of the nucleic acid present in the sample. The presence and/or amount of the nucleic acid following nucleic acid amplification can be determined by techniques well-known for the skilled person. Amplification products may e.g. be detected via labels, e.g. fluorescent labels, that are directly incorporated in the amplification product. Such labels are typically incorporated by the use of labelled oligonucleotides, e.g. primers. It will be appreciated by those skilled in the art that determining a value indicative for the presence and/or amount of the nucleic acid present in the sample may involve the addition of further reagents, such as a hybridization buffer.

For instance, the amplification product may be detected using a DNA binding dye which emits a corresponding fluorescence signal upon interaction with the double-stranded nucleic acid after excitation with light of a suitable wavelength. For example, the dyes SybrGreen and SybrGold (Molecular Probes) have proven to be particularly suitable for this application. Intercalating dyes, such as ethidium bromide, can alternatively be used.

Visualization of the amplicon may occur via agarose gel electrophoresis, which allows the determination of the presence and the size of the amplification product. The amount of the nucleic acid present in the sample may also be determined by quantitative amplification methods. Quantification of the amplification product may occur during the exponential or linear amplification phase or after completion of the amplification, e.g. via end-point detection. A typical example for quantification during amplification is RT-PCR, which allows for detection and quantification of the amplification product in real-time. Detection and quantification in RT-PCR includes the use of intercalating fluorescent dyes the use of sequence-specific labels.

Detection can be performed in a detection chamber by techniques well-known for the skilled person.

In some embodiments, the nucleic acid is indicative for a disease selected from the group consisting of a respiratory disease, COVID-19, a gastrointestinal disease and sepsis. In a specific embodiment, the nucleic acid is indicative for a respiratory disease. In another specific embodiment, the nucleic acid is indicative COVID-19. In another specific embodiment, the nucleic acid is indicative for a gastrointestinal disease. In another specific embodiment, the nucleic acid is indicative for sepsis.

In some embodiments, one or more of the above-described steps are performed in a microfluidic cartridge.

The following examples are put forth so as to provide those of ordinary skill in the art with a complete disclosure and description of how to make and use the present disclosure, and are not intended to limit the scope of what the inventors regard as their invention.

### EXPERIMENTAL SECTION

### Example 1. Calculations of potential nucleic acid amplification in the presented methods.

In the present calculations, a sample volume (110 µl) exceeding the capabilities of a single amplification reaction is subjected as aliquots to 10 pre-amplification steps (N = 8). Each pre-amplification step contains 5 PCR cycles with reaction volume of 20 µl, followed by 45 main amplification cycles. Perfect exponential (2n) amplification efficiency for the pre-amplification steps is assumed in the calculations. It is also assumed that there is only one amplifiable molecule (e.g. a target nucleic acid) in the total sample volume comprising a mix of necessary reagents for the PCR reaction and the target nucleic acid molecule.

The calculation was performed using 5 PCR cycles in each pre-amplification step and having 50% of the reaction volume of 20 µl (e.g. 10 µl) replaced with the remaining reaction mixture between pre-amplification steps. The calculated amounts of amplicons (e.g. template molecules) are listed in Table 1.

From Table 1, it can be seen that no matter in which of the 10 fractions of the 110 µl of reaction mixture volume the single template molecule is introduced to the pre-amplification process, cumulative amplification can provide enough material for the final PCR amplification for true positive detection.

Comparison of theoretical single molecule sensitivity in the amplification method according to the present disclosure and regular PCR (with a volume of the reaction chamber of 20 µl): in a regular PCR with the aforementioned parameters, there would be only 20/110 = 18% chance of getting true positive result. In other words, in most cases, the regular PCR would have given a false negative result due to lack of sufficient amount of template molecules to be introduced to the PCR reaction.

### Example 2. A cumulative PCR (cPCR) assay with a blood sample derived eluate potentially comprising sepsis bacteria

Volume of 310 µl of an eluate derived from a blood sample is mixed with master mix (primers, polymerase enzyme, nucleotides and buffer components) for a cumulative PCR reaction so that the eluate content in total reaction mixture is 40%. This premixed reaction solution of 750 ul is subjected to 6 pre-amplification cycles as aliquots in two PCR chambers (11/chamber). After the 6 cycles of amplification for the initial 20 µl aliquot, the PCR chamber is further filled with 20 µl aliquot of the premixed reaction solution. The fraction of the first aliquot moves forward from the PCR chamber to the waste reservoir while filling the reaction chamber with new aliquot. The 10 refills have a volume of 20 µl each. During the process of inserting a filling aliquot to the reaction chamber, a corresponding volume of liquid is moved from the reaction chamber to the waste reservoir. The refill of 20 µl to 20 µl PCR chamber does not completely remove previous volume in the chamber but instead part of the volume with possible pre-amplified amplicons will stay and be subjected to next pre-amplification cycles.

The change ratio of the amplified reaction mixture present in the reaction chamber to the aliquot volume used for refilling have been determined using Comsol simulation. With 7 µl filling, no loss of refill volume occurs by liquid going through the PCR chamber. With 20 µl refill, part of added refill goes through the chamber already during the filling step. However, our results confirmed that the described filling procedure ensures that a maximum amount of reaction solution is processed in PCR and sufficient amount of amplification products produced in any stage of the pre-amplifications steps are carried to the next pre-amplification step and to the final amplification required for a subsequent detection step.

The whole cPCR process is run on a Novodiag cartridge (Mobidiag, Espoo, Finland) and detected by hybridization in Novodiag instrument (Mobidiag, Espoo, Finland) (see Figure 2). The detection can also be done in qPCR in the Novodiag instrument (see Figure 1 as an example).

### Example 3: Comparison of an exemplary method of the present disclosure with conventional qPCR for the detection of a low copy number nucleic acid present in a dilute sample.

### Introduction

The aim of this example is to compare the performance of an exemplary method of the present disclosure with a conventional qPCR approach to detect the presence of a target nucleic acid present at low concentration in a sample with large volume. A sample was prepared to contain *Listeria monocytogenes* genomic DNA at a concentration of 50 copies per ml (cp/ml). Prior to loading into the reaction chamber, the sample was further pre-mixed with amplification reagents in a 1:1 ratio, resulting in a total sample volume of 300 µl. Thus, in the total sample volume of 300 µl, approximately 7.5 copies of genomic DNA from *L. monocytogenes* were present. Using the method of the present disclosure, a total of 221 µl of the total sample volume were subjected to amplification, while 20 µl of the total sample volume, e.g. the volume of a typical reaction chamber, were subjected to conventional qPCR. To determine a positivity rate for the detection of the target nucleic acid and to compare the performance of the two methods, the method of the present disclosure was run in 8 replicates (8 cartridges of the present disclosure were used) and qPCR was run in 90 replicates.

### Methods

### Exemplary method of the present disclosure.

In the first step of a method of the present disclosure, a first reaction mixture was provided in the reaction chamber comprising 29 µl of the sample (= first sample portion to fill the chamber (20 µl) and channel prior it). Subsequently, the first reaction mixture was subjected to a first pre-amplification of 4 cycles, with each cycle comprising denaturation at 109°C for 15 seconds followed by 25 seconds of annealing at 57°C. In a third step, a second reaction mixture was provided by transferring a further sample portion of 12 µl into the reaction chamber while a similar volume of roughly 12 µl of the first reaction mixture was removed from the reaction chamber. Thus, the second reaction mixture consisted of approximately 12 µl of a second sample portion and 8 µl of the first reaction mixture. In a fourth step, the second reaction mixtures was subjected to a second pre-amplification using the same cycling conditions as described above. Step 3 and 4 are repeated 14 times resulting in a 16^{th} pre-amplification mixture. Prior to the amplification step, roughly 12 µl of the 16^{th} pre-amplification mixture were removed by introducing a further sample portion of 12 µl into the reaction chamber. Thus, prior to the final amplification, a total sample portion of 221 µl were subjected to a total of 16 pre-amplification steps. The subsequent amplification was conducted using 25 cycles, each consisting of denaturation at 109°C for 15 seconds followed by 25 seconds of annealing at 57°C.

The presence of the target nucleic acid in the sample was further determined by subjecting the amplification reaction mixture to a hybridization step. The protocol is summarized in Table 2.

*Conventional qPCR.* 20 µl of the sample were analyzed by Biorad CFX qPCR.

### Results

As shown in Figure 4, 6 out of 8 cartridges showed positive detection of *L. monocytogenes,* i.e. the positivity rate for the exemplary method of the present disclosure was 75%. In comparison, 19 out of 90 qPCRs detected the presence of *L. monocytogenes,* i.e. the positivity rate for qPCR detection was only 21.1% (Figure 5).

### Conclusions

The method of the present disclosure allows an increased sample volume to be subjected to amplification. Here, 110.5 µl of initial sample containing approximately 5.5 copies of the target nucleic acid were run in a single protocol and the target nucleic acid was detected with 75% probability. In contrast, the reaction volume of a conventional qPCR is smaller. Here, 10 µl of the initial sample containing approximately 0.5 copies of the target nucleic acid can be run in a qPCR reaction, which resulted in a probability of detection of 21.1 %. To summarize, the probability of amplification and detection of a target nucleic acid at a concentration of 50 cp/ml in a sample volume of 150 µl was more than 3 times higher by using the exemplary method of the present disclosure compared to a conventional qPCR approach.

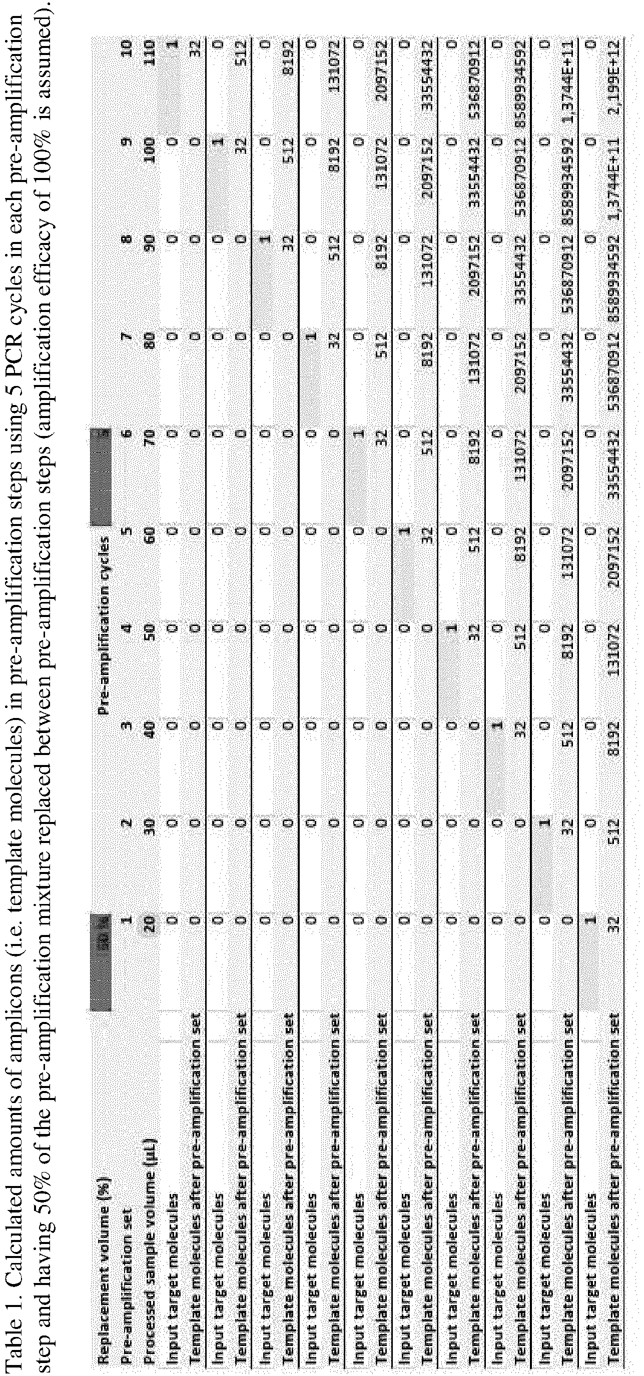

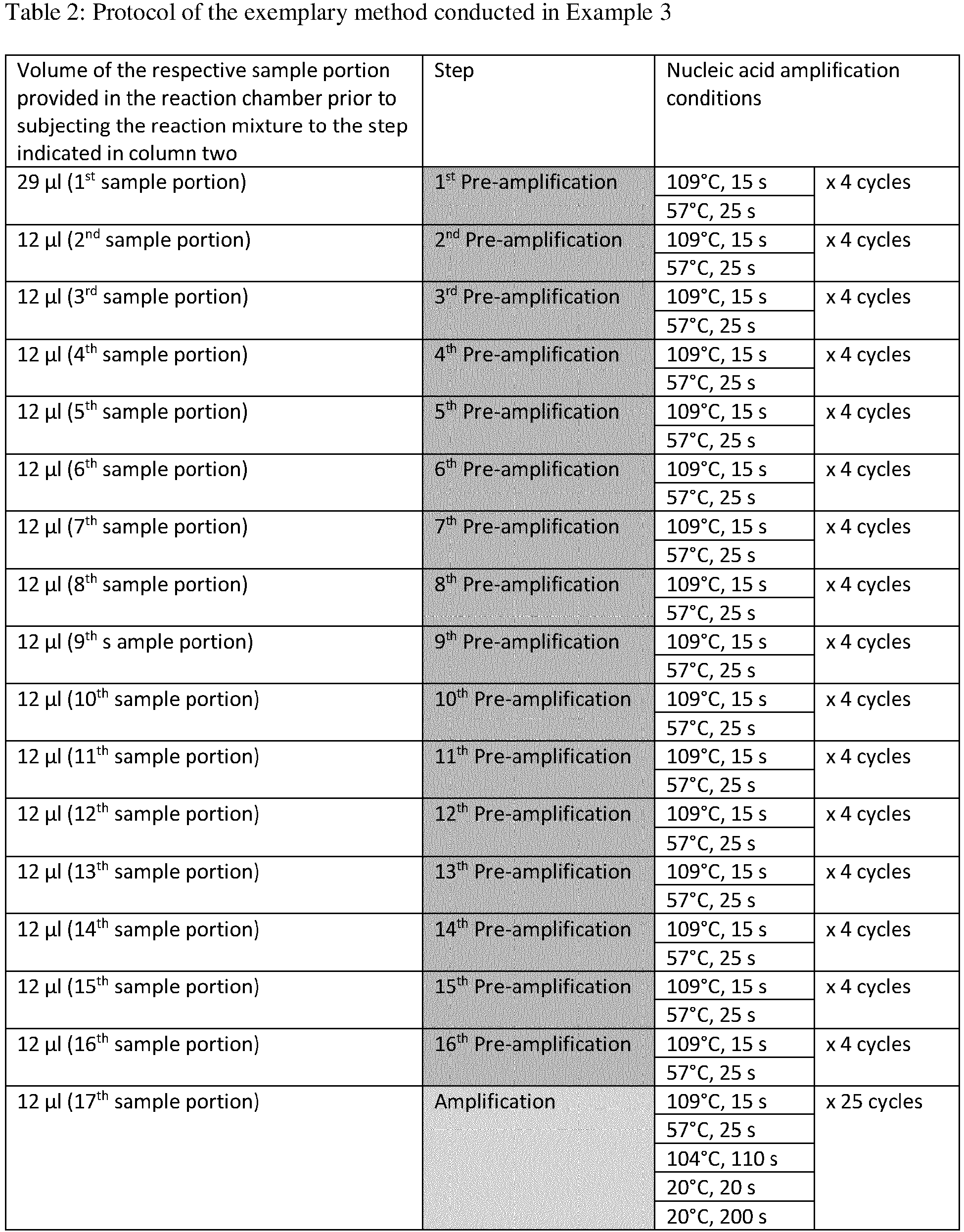

## Claims

1. A method for amplifying a nucleic acid which is suspected to be present in a sample in a low copy number, wherein the method comprises the following steps:
i. providing a first reaction mixture in a reaction chamber wherein the first reaction mixture comprises a first portion of the sample;
ii. subjecting the first reaction mixture in the reaction chamber to conditions allowing a pre-amplification of the nucleic acid in order to obtain a first pre-amplification mixture;
iii. providing a second reaction mixture in the reaction chamber wherein the second reaction mixture comprises a subset of the first pre-amplification mixture and a second portion of the sample;
iv. subjecting the second reaction mixture to conditions allowing a pre-amplification of the nucleic acid in order to obtain a second pre-amplification mixture;
v. optionally, repeating steps iii) and iv) a number N additional times in order to obtain an (N+2) pre-amplification mixture,
vi. subjecting the second pre-amplification mixture obtained in step (iv) or the (N+2) pre-amplification mixture obtained in step (v) to conditions allowing an amplification of the nucleic acid in order to obtain an amplification mixture,
wherein the sample has a total sample volume which exceeds a volume capacity of the reaction chamber.

2. The method of claim 1, wherein providing a second reaction mixture in the reaction chamber in step (iii) comprises displacing a subset of the first pre-amplification mixture in the reaction chamber by introducing the second portion of the sample into the reaction chamber.

3. The method of claim 2, wherein a volume of the second portion of the sample introduced into the reaction chamber is 1-99% of the volume of the first pre-amplification mixture, optionally 10-95%, 20-90%, 30-80% or 40 to 70% or 50-60% of the volume of the first pre-amplification mixture.

4. The method of claim 1, wherein providing a second reaction mixture in the reaction chamber in step (iii) comprises removing a subset of the first pre-amplification mixture from the reaction chamber in order to provide a remaining subset of the first pre-amplification mixture in the reaction chamber and combining the remaining subset of the first pre-amplification mixture with a second portion of the sample in order to provide the second reaction mixture.

5. The method of claim 4, wherein a volume of the subset of the first pre-amplification mixture removed in step (iii) is 1-99% of the volume of the pre-amplification mixture, optionally 25-95%, 30-90%, 40-80%, 50-70% or 55-65% of the volume of the pre-amplification mixture.

6. The method of claim 1, wherein providing a second reaction mixture in the reaction chamber in step (iii) comprises removing a subset of the first pre-amplification mixture from the reaction chamber, combining the removed subset of the first pre-amplification mixture with the second portion of the sample in order to provide a combined pre-amplification sample mixture and introducing a portion of the combined pre-amplification sample mixture into the reaction chamber in order to provide the second reaction mixture.

7. The method of claim 6, wherein a volume of the subset of the first pre-amplification mixture removed in step (iii) is 1-100% of the volume of the pre-amplification mixture, optionally 25-95%, 30-90%, 40-80%, 50-70% or 55-65% of the volume of the pre-amplification mixture.

8. The method of any of the preceding claims, wherein the total sample volume exceeds the volume capacity of the reaction chamber by at least 110%, optionally at least 150%, at least 200% or at least 1000%.

9. The method of any of the preceding claims, wherein the volume capacity of the reaction chamber is 10-70 µl, optionally 20-50 µl or 30-40 µl.

10. The method of any of the preceding claims, wherein the total sample volume is 100-500 µl, optionally 150-450 µl or 200-400 µl or 250-350 µl or about 300 µl.

11. The method of any of the preceding claims, wherein the average amount of nucleic acid in the sample is less than 25 copies per 50 µl, optionally less than 15 copies or less than 5 copies per 50 µl.

12. The method of any of the preceding claims, wherein a volume of the first reaction mixture of step (i) is substantially identical to a volume of the second reaction mixture of step (iii).

13. The method of any of the preceding claims, wherein
- both the pre-amplification and the amplification is a cyclic amplification, or
- both the pre-amplification and the amplification is an isothermal amplification, or
- the pre-amplification is a cyclic amplification and the amplification is an isothermal amplification, or
- the pre-amplification is an isothermal amplification and the amplification is a cyclic amplification.

14. The method of any of the preceding claims, wherein the pre-amplification is a cyclic amplification with 3-15 cycles, optionally 3-12 cycles or 4-11 cycles or 4-10 cycles or 4-8 cycles or 4-6 cycles.

15. The method of any of claims 1-12, wherein the pre-amplification is an isothermal amplification of 5 to 30 min.

16. A method of any of the preceding claims, wherein the amplification in step (vii) is a cyclic amplification having 50 cycles or less, optionally 25-45 cycles or optionally 30 to 40 cycles or about 35 cycles.

17. A method of any of claims 1-15, wherein the amplification in step (vii) is an isothermal amplification of 20 to 60 min.

18. The method of any of the preceding claims, wherein N is sufficient to subject substantially the total sample volume to conditions allowing a pre-amplification of the nucleic acid.

19. The method of any of the preceding claims, wherein the first and second reaction mixture comprise one or more of a DNA-dependent DNA polymerase, a DNA-dependent RNA polymerase, and an RNA-dependent DNA polymerase.

20. The method of claim 19, wherein the first and second reaction mixture comprise a DNA-dependent DNA polymerase selected from a *Taq* polymerase, SuperFi DNA Polymerase, AccuPrime *Pfx* DNA Polymerase, *Pfu* polymerase, deep vent polymerase, *Pwo* polymerase, *Tli* polymerase, *Tfu* polymerase, T4 DNA polymerase, T7 DNA polymerase, and Q5 DNA polymerase.

21. The method of claim 19, wherein the first and second reaction mixture comprise a DNA-dependent RNA polymerase selected from a T7 RNA Polymerase; T3 RNA Polymerase, SP6 RNA Polymerase, Poly (A)- or Poly (U)-polymerase, and Hi-T7 RNA Polymerase.

22. The method of claim 19, wherein the first and second reaction mixture comprise a RNA-dependent DNA polymerase selected from a Moloney Murine Leukemia Virus Reverse Transcriptase (M-MLV RT), Avian Myeloblastosis Virus Reverse Transcriptase (AMV RT), ProScript Reverse Transcript, and SuperScript reverse transcriptase.

23. The method of any of the preceding claims, wherein subjecting to conditions allowing an amplification comprises adding further amplification reagents wherein said further amplification reagents comprise one or more of a DNA-dependent DNA polymerase, a DNA-dependent RNA polymerase, and an RNA-dependent DNA polymerase.

24. A method of claim 23, wherein the added amplification reagents comprise a DNA-dependent DNA polymerase selected from a *Taq* polymerase, SuperFi DNA Polymerase, AccuPrime *Pfx* DNA Polymerase, *Pfu* polymerase, deep vent polymerase, *Pwo* polymerase, *Tli* polymerase, *Tfu* polymerase, T4 DNA polymerase, T7 DNA polymerase, and Q5 DNA polymerase.

25. A method of claim 23, wherein the added amplification reagents comprise a DNA-dependent RNA polymerase selected from a T7 RNA Polymerase; T3 RNA Polymerase, SP6 RNA Polymerase, Poly (A)- or Poly (U)-polymerase, and Hi-T7 RNA Polymerase.

26. A method of claim 23, wherein the added amplification reagents comprise a RNA-dependent DNA polymerase selected from a Moloney Murine Leukemia Virus Reverse Transcriptase (M-MLV RT), Avian Myeloblastosis Virus Reverse Transcriptase (AMV RT), ProScript Reverse Transcript, and SuperScript reverse transcriptase.

27. The method of any of the preceding claims, comprising a further step: (vii) determining in the amplification reaction mixture obtained in step (vi) a value indicative for the presence and/or amount of the nucleic acid present in the sample.

28. The method of any of the preceding claims, wherein the nucleic acid is indicative for a disease selected from the group consisting of a respiratory disease, COVID-19, a gastrointestinal disease and sepsis.

29. The method of any of the preceding claims wherein one or more of steps (i) to (vii) and optionally (viii) are performed in a microfluidic cartridge.

30. A method for amplifying a nucleic acid which is suspected to be present in a sample in a low copy number, wherein the method comprises the following steps:
i. providing a first reaction mixture in a reaction chamber wherein the first reaction mixture comprises a first portion of the sample;
ii. subjecting the first reaction mixture in the reaction chamber to conditions allowing a pre-amplification of the nucleic acid in order to obtain a first pre-amplification mixture;
iii. removing a subset of the first pre-amplification mixture from the reaction chamber in order to provide a remaining subset of the first pre-amplification mixture in the reaction chamber;
iv. providing a second reaction mixture in the reaction chamber wherein the second reaction mixture comprises the remaining subset of the first pre-amplification mixture and a second portion of the sample;
v. subjecting the second reaction mixture to conditions allowing a pre-amplification of the nucleic acid in order to obtain a second pre-amplification mixture;
vi. optionally, repeating steps iii) to v) a number N additional times in order to obtain an (N+2) pre-amplification mixture,
vii. subjecting the second pre-amplification mixture obtained in step (v) or the (N+2) pre-amplification mixture obtained in step (vi) to conditions allowing an amplification of the nucleic acid in order to obtain an amplification mixture,
wherein the sample has a total sample volume which exceeds a volume capacity of the reaction chamber.

31. A method for amplifying a nucleic acid which is suspected to be present in a sample in a low copy number, wherein the method comprises the following steps:
i. providing the sample in a sample chamber;
ii. transferring a first portion of the sample to a reaction chamber in order to obtain a first reaction mixture;
iii. subjecting the first reaction mixture in the reaction chamber to conditions allowing a pre-amplification of the nucleic acid in order to obtain a first pre-amplification mixture;
iv. transferring at least a subset of the first pre-amplification mixture from the reaction chamber to the sample chamber comprising a second portion of the sample in order to provide a combined pre-amplification sample mixture;
v. transferring a portion of the combined pre-amplification sample mixture to the reaction chamber in order to provide the second reaction mixture
vi. subjecting the second reaction mixture to conditions allowing a pre-amplification of the nucleic acid in order to obtain a second pre-amplification mixture;
vii. optionally, repeating steps iii) and iv) a number N additional times in order to obtain an (N+2) pre-amplification mixture,
viii. subjecting the second pre-amplification mixture obtained in step (v) or the (N+2) pre-amplification mixture obtained in step (vi) to conditions allowing an amplification of the nucleic acid in order to obtain an amplification mixture,
wherein the sample has a total sample volume which exceeds a volume capacity of the reaction chamber.

32. A method for amplifying a nucleic acid which is suspected to be present in a sample in a low copy number, wherein the method comprises the following steps:
v. providing a first reaction mixture in a reaction chamber wherein the first reaction mixture comprises a first portion of the sample;
vi. subjecting the first reaction mixture in the reaction chamber to conditions allowing a pre-amplification of the nucleic acid in order to obtain a first pre-amplification mixture;
vii. providing a second reaction mixture in the reaction chamber wherein the second reaction mixture comprises a subset of the first pre-amplification mixture and a second portion of the sample;
viii. subjecting the second reaction mixture to conditions allowing an amplification of the nucleic acid in order to obtain an amplification mixture,
wherein the sample has a total sample volume which exceeds a volume capacity of the reaction chamber.
